# EUROPEAN PATENT APPLICATION

(11) **EP 2 607 478 A1**
(43) Date of publication of application: **26.06.2013**
(21) Application number: 11194815.4
(22) Date of filing: 21.12.2011
(51) Int. Cl.: C12N 5/0775, A61K 35/28, A61K 31/435

(54) **Phenanthroline treated MSC**

(71) Applicant: Universiteit Twente, 7522 NB Enschede (NL)
(72) Inventor: Fernandes, Hugo Agostinho Machado, 7522 NB Enschede (NL); de Boer, Jan, 7522 NB Enschede (NL); Quax, Paulus Hubertus Andreas, 7522 NB Enschede (NL); Doorn, Joyce, 7522 NB Enschede (NL)
(74) Representative: Jansen, Cornelis Marinus

(57) **Abstract**

The invention relates to the field of medicine and in particular to the treatment of ischemia and to angiogenesis. The invention further relates to novel uses of phenanthroline in replacing a missing biological structure, supporting a damaged biological structure and/or enhancing an existing biological structure and to novel medical products.

SEQUENCE LISTING

<110>  Universiteit Twente

<120>  Phenanthroline treated MSC

<130>  P96223EP00

<140>  EP 1194815.4
<141>  2011-12-21

<160>  2

<170>  PatentIn version 3.3

<210>  1
<211>  20
<212>  DNA
<213>  Artificial

<220>
<223>  primer

<400>  1
cggctaccac atccaaggaa                                                    20


<210>  2
<211>  18
<212>  DNA
<213>  Artificial

<220>
<223>  primer

<400>  2
gctggaatta ccgcggct                                                      18

## Description

The invention relates to the field of medicine and in particular to the treatment of ischemic disease and to improving angiogenesis. The invention further relates to a method of culturing MSC, novel uses of phenanthroline in replacing a missing biological structure, supporting a damaged biological structure and/or enhancing an existing biological structure, and to novel medical products.

Angiogenesis, the formation of new blood vessels, is a physiological and vital process in growth and development, as well as in wound healing. Wounds that are poorly vascularised and/or ischemic wounds heal slower, and research has thus been directed towards inducing angiogenesis in, for instance, ischemic chronic wounds.

In contrast to the physiological and typically beneficial role, angiogenesis may also play a role in pathological processes, such as cancer.

Thus, whereas anti-angiogenic therapies are generally being employed to fight cancer and malignancies, pro-angiogenic therapies are generally explored as options to treat for instance cardiovascular diseases, graft survival, and wound healing. Clinical research in therapeutic angiogenesis is ongoing for a variety of medical conditions. Presently, three main categories of angiogenic methods are explored: gene therapy, protein therapy, and cell-based therapy.

There are, however still unsolved problems associated with angiogenic therapy. Gene therapy is, for instance, hampered by difficulties in effective integration of the therapeutic genes into the genome of target cells, the risk of an undesired immune response, potential toxicity, and oncogenesis related to the viral vectors used in gene therapy. It is further questioned whether a single gene may be effective in restoring the defects in diseases that are multifactorial, such as diabetes or heart disease. Cell-based angiogenic therapy, is still in an early stage of research, with questions regarding the cell type to be used, obtaining the desired cell-type and post-transplant survival of the transplanted cells.

There is thus need for an alternative angiogenic therapy. An objective of the invention is to provide means and methods for such alternative therapy.

In one embodiment, the invention provides a method for culturing mesenchymal stromal cell (MSC), the method comprising
- providing a MSC; and
- culturing said MSC in the presence of 1, 10-phenanthroline or an active derivative thereof.

The invention provides the insight that a MSC, cultured in the presence of 1, 10-phenanthroline or an active derivative thereof, but also a supernatant thereof, is for instance useful as a medicament for, amongst others, the treatment of ischemia and/or for inducing angiogenesis.

In a preferred embodiment, a method according to the invention is provided, the method further comprising the step of:
- harvesting the thus cultured cells and/or harvesting a supernatant thereof.

A mesenchymal stromal cell (MSC; synonyms: mesenchymal stem cell, marrow stromal cell, or multipotent stromal cell) is a multipotent stem cell, which means that it is generally able to differentiate into different cell-types, typically including osteoblasts (bone cells), chondrocytes (cartilage cells) and adipocytes (fat cells). However, a MSC is typically not omnipotent, i.e. it does not have an unlimited repertoire. For instance, a MSC typically does not differentiate into a cell of the myeloid lineage. MSC are present in many different tissues of the (human) body. A MSC can, for instance, be obtained by bone marrow puncture, from adipose tissue, tendons, ligaments, umbilical cord blood, Wharton's Jelly, dental pulp, etc.

The International Society for Cellular Therapy has proposed the following minimal criteria for defining multipotent MSC (25):
First, according to these minimal criteria, MSC is plastic-adherent when maintained under standard culture conditions.
Second, MSC express CD105 and CD90 and preferably also expresses CD73. MSC typically lack or are low for expression of CD45, CD34, CD11b, CD19 and HLA-DR surface molecules and preferably also lack or are low for expression of CD14 or CD79alpha.
Third, MSC are able to differentiate to osteoblasts, adipocytes and chondroblasts *in vitro.* In a preferred embodiment, a method according to the invention is provided, wherein said a MSC is used, which is capable of adhering to plastic when maintained under standard culture conditions. In a more preferred embodiment, a method according to the invention is provided, wherein said MSC expresses CD105, CD73, and CD90 and lacks expression of CD45, CD34, CD14 or CD11b, CD79alpha or CD 19, and HLA-DR surface molecules. Preferably, said MSC is capable of differentiating to osteoblasts, adipocytes and chondroblasts *in vitro.*

MSC can be obtained from a variety of different tissues. Sources for MSC include bone marrow, trabecular bone, periosteum, articular cartilage, synovium, synovial fluid, muscle, adipose tissue, tendons, ligaments, blood, blood vessels, umbilical cord vasculature, fetal tissue, skin, spleen, and thymus (59-61). In a preferred embodiment, a method according to the invention is provided, wherein the MSC is obtained from bone marrow, tendon, ligament, bone, or cartilage, more preferably from tendon or ligament. MSC are easily obtained from tendon or ligament, for instance by performing a biopsy. Such ligament- or tendon-derived MSC, reviewed by Lui and Chan (60), are especially useful in medical applications directed to tendon or ligament repair, but may also be used for other medical applications.

The term "culturing" is herein defined as maintaining MSC under suitable conditions, i.e. conditions that typically allow a MSC to survive. Generally MSC are cultured in in a specially prepared nutrient medium at 37°C. However, for shorter periods of time, it is also possible to culture MSC for instance in phosphate buffered saline, preferably comprising nutrients such as glucose and minerals, or at lower temperatures. A person skilled in the art can easily determine suitable conditions for culturing MSC for a predetermined period of time.

The term "supernatant" is herein defined as a liquid present above a (cell) sediment or a (cell) layer, said sediment or layer preferably comprising a cell cultured in the presence of 1, 10-phenanthroline or an active derivative thereof, more preferably comprising a MSC cultured in the presence of 1, 10-phenanthroline or an active derivative thereof. In case of, for instance, an adherent cell (i.e. a cell that has adhered to a surface, typically a surface of, for instance, a cell flask, petri-dish, or culture plate), the liquid that covers the cell during culture is the supernatant. Typically, supernatant is a culture medium, preferably a pharmaceutically acceptable culture medium. However, especially for short incubation times, physiologic saline or a buffered saline, is equally suitable. Any other liquid, suitable for culturing a cell, preferably a MSC can be used in a method of the invention. In a preferred embodiment, a method according to the invention is provided, wherein the supernatant is a (buffered) saline solution or a culture medium, preferably a culture medium, more preferably a pharmaceutically acceptable culture medium.

The term "harvesting" is herein defined as the act or process of gathering (a) cell(s) or a supernatant from a cell culture. Preferably, the cells cultured using a method according to the invention are harvested alive, i.e. in a vital state. Harvesting of cultured cells can be done by a procedures known in the art, such as by using trypsine, a rubber policeman, or a combination thereof. For harvesting a supernatant of the invention, it is not necessary that the cultured cells survive, although it is preferred that the cells are not (extensively) damaged during the harvesting step. When cells are damaged, they may release products, such as intracellular proteases, which may influence the quality of the supernatant. Typically supernatant is harvested by decanting supernatant of a cell layer or pellet, optionally after a centrifugation step.

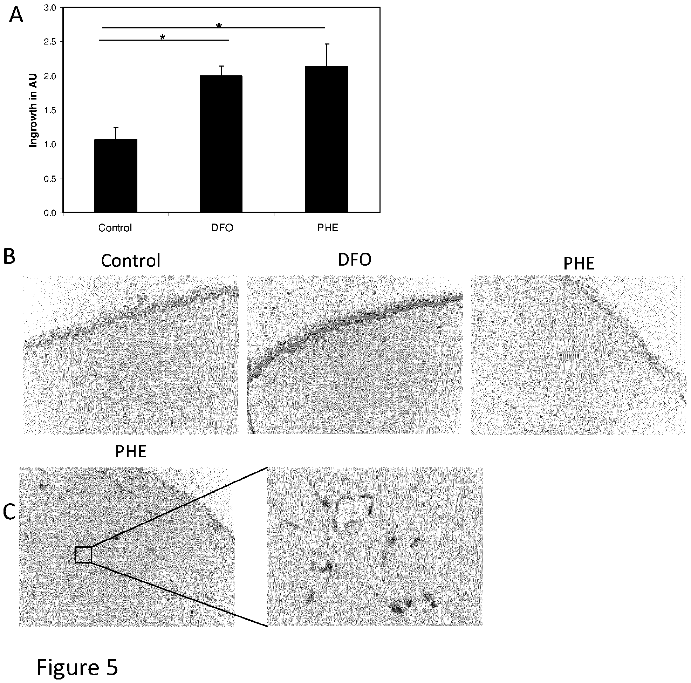

1, 10-phenanthroline, further referred to as "phenanthroline", is a hetero-aromatic compound having a formula as depicted in Figure 5. Phenanthroline is capable of inducing HIF-1alpha activity in an human cervical cancer cell line (28). Activation of HIF-1alpha activity is thought to be a result of the metal chelating properties of phenanthroline. It is further thought that phenanthroline inhibits degradation of HIF-1alpha by virtue of chelation of the metal ion required for catalytic activity of the proteolytic enzyme prolyl hydroxylase (58).

The term "active derivative" is herein defined as a compound having structural similarities, preferably having a substituted backbone of phenanthroline. Preferably, an active derivative of phenanthroline has the same effect on MSC, in kind, not necessarily in amount as 1,10-phenanthroline.

It is for instance generally possible to add substitutions to any one of the carbon atoms at the 2, 3, 4, 5, 6, 7, 8 and/or 9 position, without substantially losing the metal chelating properties of the phenanthroline backbone (23, 24).

Non-limiting examples of substituted phenanthroline derivatives are depicted in Figure 5. In addition to the substituents depicted in Figure 5, other chemical groups, including sulfur, or amino substitutions and combinations of any of the substituents are possible. In a preferred embodiment, a method according to the invention is provided, wherein the active derivative has the same metal chelating activity, in kind, not necessarily in amount as phenanthroline. Preferably the active phenanthroline derivative is substituted on the 2, 3, 4, 5, 6, 7, 8, and/or 9 position, more preferably on the 3, 4, 5, 6, 7, and/or 8 position, more preferably on the 4, 5, 6, and/or 7 position, most preferably on the 5 and/or 6 position. Preferably a substitution reduces hydrophobicity, such that the compound is more easily soluble in water, or is more stable in water.

It is also possible to use a derivative of phenanthroline substituted with a chemical reactive group, such as for instance a sulfide, or a benzylic halogen. Such substituted phenanthroline is very useful for coupling to a matrix or a gel or any other material for use in the invention. As implied in the term "active derivative", the thus coupled compound preferably still has the same activity, in kind, not necessarily in amount, as phenanthroline. Preferably, the thus coupled compound has the same metal chelating properties, in kind, not necessarily in amount, as phenanthroline.

In a preferred embodiment, a method according to the invention is provided, wherein said active derivative of phenanthroline, is a phenanthroline compound substituted at the 2, 3, 4, 5, 6, 7, 8, and/or 9 position, preferably at the 3, 4, 5, 6, 7, and/or 8 position, more preferably at the 4, 5, 6, and/or 7 position, most preferably at the 5 and/or 6 position. Preferably the substituent(s) are chosen, independently from one another from NO, OH, CH3, O, S, Br, Cl, and I. When it is advantageous to reduce hydrophobicity, the substituent(s) are preferably chosen, independently from one another from NO, OH, O, and S. When substitution is performed in order to enable the compound to be coupled with for instance a matrix or a gel, a substituent is preferably chosen from S, Br, Cl, and I.

MSC generally secrete a broad panel of growth factors and cytokines that have trophic properties (i.e. anti-apoptotic and anti-inflammatory) (1). Several clinical trials have been completed or are currently on the way investigating the use of MSCs for the treatment of, amongst others, autoimmune diseases (2-5), myocardial infarcts [reviewed in (6)], solid organ/graft transplantations (7, 8) and ischemic wounds (9). Although differentiation of MSCs into cells of the target tissue has been shown (10-12), the use of MSC for use in the above conditions generally results in low engraftment percentages and there is only a short window in which effects are observed (13-16). The present invention now provides a method for treating MSC such, that the resulting MSC and supernatant thereof have improved properties, such as for instance improved angiogenic properties and/or improved graft survival.

In a preferred embodiment, a method for manufacturing phenanthroline treated MSC and/or supernatant thereof is provided, for improving an angiogenic capacity of a MSC and/or a supernatant thereof. Said method preferably resulting in a collection of cells and/or supernatant thereof with improved angiogenic capacity.

It is preferred to use primary MSC in a method of the invention, especially when the MSC themselves are to be used for treating a subject. Preferably, a MSC used in a method according to the invention is obtained from the same subject that is to be treated with a cell and/or supernatant obtained by a method according to the invention. This is called an autologous transfusion. An autologous transfusion typically bears less risk of adverse reactions, such as for instance a graft versus host reaction, than a heterologous transfusion (i.e. obtained from one subject and transfused to another subject).

In a preferred embodiment, a method according to the invention is provided, wherein the MSC for use in the method is a primary cell, preferably a human primary MSC. In another preferred embodiment, a MSC for use in a method according to the invention is an immortalized MSC. Especially when supernatant is used, and not the cultured cells, it is preferred to use immortalized MSC in a method according to the invention. Immortalized MSC are generally more easily cultured and maintained than primary cells and typically secrete a similar cytokine/growth factor pattern. Supernatant from immortalized cells is thus also suitable for treating a subject. When it is envisaged to treat a human subject with cells and/or supernatant obtained by the method of the invention, a MSC to be used in the method is preferably a human MSC. In a preferred embodiment, a method according to the invention is provided, wherein the MSC is a primary MSC, preferably a primary human MSC.

In another preferred embodiment, a method according to the invention is provided, wherein the MSC is an immortalized MSC, preferably an immortalized human MSC.

The invention shows in a working example that cells which are cultured for about 48 hours at a density of about 10,000 cells / cm² with a concentration of about 200 µM phenanthroline show desirable characteristics. However, the skilled person is quite capable of adapting these parameters for specific needs and for instance by increasing the cell density and phenanthroline concentration or active derivative of phenanthroline, and at the same time decreasing contact time, achieve similar results. In particular, concentrations of phenanthroline or an active derivative thereof are used that show a desired effect (e.g. improved angiogenic capacity), without significantly inducing apoptosis.

In a preferred embodiment, a method according to the invention is provided, wherein the concentration of phenanthroline or active derivative of phenanthroline in the culture is not more than 1 mM and not less than 1 uM. Preferably the concentration of phenanthroline or active derivative of phenanthroline is between 10 uM and 1000 uM, more preferably between 20 uM and 500 uM, more preferably between 50 uM and 300 uM, more preferably between 100 uM and 200 uM, most preferably between 150 and 200 uM.

In a preferred embodiment, a method according to the invention is provided, wherein said MSC are contacted with said phenanthroline or an active derivative thereof at a cell density of at least 1,000 cell/cm2. Preferably, the cells are contacted at a cell density of lower than 50,000 cells/cm2, more preferably between 2,000 - 20,000 cells/cm2, more preferably between 5,000 - 15,000 cells/cm2, most preferably at about 10,000 cells/cm2.

In a preferred embodiment, a method according to the invention is provided, wherein the duration of contacting said MSC with phenanthroline or the active derivative thereof is for at least 12 hours. Preferably, the MSC are contacted with phenanthroline or an active derivative thereof for not more than 96 hours. More preferably, the MSC are contacted with phenanthroline or an active derivative thereof for between 24 - 72 hours, more preferably for between 36 - 60 hours, most preferably for about 48 hours.

Now that the invention provides a method for culturing MSC in the presence of phenanthroline or active derivative of phenanthroline, the invention further provides a collection of cells or a supernatant obtainable by such method.

In one embodiment, the invention provides a collection of cells and/or a supernatant obtainable by a method, the method comprising
- providing a MSC;
- culturing said MSC in the presence of phenanthroline or an active derivative thereof; and
- harvesting the thus cultured cell(s) and/or supernatant thereof.

A collection of cells obtainable by a method according to the invention are distinguishable from other collection of cells known in the art by their specific secretion pattern as described in the examples. The culture of cells for instance comprises phenantroline or active derivative of phenanthroline cultured MSC cells that express VGEF and cells that express IL-8 in an amount of at least 200 pg/ml culture supernatant per 48 hours of culture. The supernatant, comprising proteins, such as growth factors and/or cytokines, secreted by the phenanthroline or active derivative thereof cultured MSC, is thus also distinguishable from supernatants described before.. The supernatant for instance comprises supernatant from phenantroline or active derivative of phenanthroline cultured MSC cells that comprises excreted VGEF and excreted IL-8 in an amount of at least 200 pg/ml culture supernatant.

As said above, a cell obtainable by a method according to the invention preferably secretes different growth factors and/or cytokines (i.e. the cell has changed its secretome) in respect to a MSC which has not been treated with phenanthroline or an active derivative thereof. A cell obtainable by a method according to the invention, may also, but not necessarily, express different cell-surface markers and, therefore, does not necessarily express all the markers, or is not necessarily deficient in expression of all the markers as defined by the International Society for Cellular Therapy.

A cell obtainable by a method according to the invention is useful as a medicament, for instance, for treatment of ischemia, for angiogenesis, in wound repair, in tendon and ligament regeneration, etc.

In one embodiment, therefore, the invention provides a collection of cells obtainable by a method according to the invention for use as a medicament. In a preferred embodiment, the MSC cultured in a method according to the invention is a huma MSC.

The invention further provides supernatant obtainable by a method according to the invention for use as a medicament. Before use as a medicament, the supernatant is preferably concentrated in order to reduce the volume and/or increase the concentration of, amongst others, growth factors and cytokines. Preferably, the supernatant is concentrated such, that the volume is decreased without substantially altering the absolute amount of proteins, especially growth factors and cytokines, present in the supernatant. It is also preferred that the concentration of proteins present in the supernatant, relative to each other, remains essentially the same. Preferably, by concentrating a supernatant of the invention, the supernatant is depleted from water, salts, glucose, and other small molecules present in the supernatant, whereas larger proteins, like VEGF and IL8 are retained. Several methods for concentrating proteins in a supernatant are known in the art and include, for instance, ultrafiltration, centrifugation (e.g. using a concentrator), freeze drying, centrifugal evaporation, etc.

In a preferred embodiment, the invention provides a cell and/or supernatant obtainable by a method according to the invention for use in the treatment of ischemia. By administering such cell or (concentrated) supernatant (or a combination thereof) to a subject suffering from ischemia, the cell or supernatant will aid to the treatment of ischemia and/or improve blood vessel formation.

In a preferred embodiment, the invention provides a cell, preferably a huma MSC, obtainable by a method according to the invention and/or a supernatant obtainable by a method according to the invention, for inducing angiogenesis. As shown by the present invention, a cell cultured in the presence of phenanthroline or an active derivative thereof, but also a growth factor, such as VEGF and/or a cytokine, such as IL8, secreted by such cultured cell, will increase angiogenic processes when administered to a subject in need of angiogenesis. By inducing angiogenesis, it is for instance achieved that collateral veins are formed, which are able to short-circuit an obstructed or insufficient vein.

It is also possible to use of a cell or a supernatant obtainable by a method according to the invention in tendon or ligament regeneration, as these biological structures are notorious for slow healing due to poor vascularisation. In a preferred embodiment, a method according to the invention is provided, wherein the MSC is obtained from ligament or tendon. It is especially useful to culture MSC obtained from ligament or tendon, when the thus cultured cells are to be used for tendon or ligament regeneration. However, cells obtained by a method according to the invention, wherein the MSC are obtained from ligament or tendon tissue, can also be used in other medical applications, such as wound repair, improving transplant survival, etc.

In a preferred embodiment, a cell or supernatant obtainable by a method according to the invention is provided, for use in tendon and/or ligament regeneration, preferably after tendon and/or ligament construction, more preferably after anterior cruciate ligament construction. A cell and/or supernatant obtainable by a method according to the invention enables improving vascularisation of the damaged tendon and/or ligament and consequently advances the healing process of said tendon and/or ligament. When the thus cultured cells or supernatant thereof are to be used in tendon and/or ligament regeneration, the method preferably comprises culturing of a ligament derived MSC or a tendon derived MSC.

Further provided is a cell or a (concentrated) supernatant, obtainable by a method according to the invention for use in wound healing. Standard treatment of (large) wounds is typically done by closing of the wound with a suture and/or a patch. A cell and/or supernatant obtainable by a method according to the invention is very useful in such situation. The wound can for instance, before closing, first be treated with a cell obtainable by a method according to the invention. Also, direct application of supernatant from such cell is possible. It is, however, also possible to close the wound with a suture and/or a patch comprising a cell obtainable by a method according to the invention. It is also possible to use a suture and/or a patch comprising a supernatant obtainable by a method according to the invention, or comprising phenanthroline or an active derivative thereof.

A cell or a (concentrated) supernatant obtainable by a method according to the present invention is also very useful for improving graft survival of for instance (allogenic) organ grafts or (allogenic) bone marrow grafts. Graft survival is significantly improved when such graft is well vascularised. In case of a bone marrow graft, a cell or a (concentrated) supernatant obtainable by a method according to the invention preferably improves survival of the transplanted bone marrow cells. Without being bound to theory, a growth factor and/or cytokine secreted by the cell and/or present in the supernatant may inhibit apoptosis of the grafted cells. Also for artificial grafts that have been shaped in vitro, such as for instance tissue engineered constructs (e.g. skin appendices, bone structures, etc.), a cell or supernatant obtainable by a method according to the invention is especially useful.

In a preferred embodiment, a cell or a supernatant obtainable by a method according to the invention is provided, for use in improving graft survival, preferably for use in improving organ or bone marrow graft survival.

In another preferred embodiment, a cell or a (concentrated) supernatant obtainable by a method according to the invention is provided, for use in the treatment of osteoporosis and/or bone fractures.

Now that the invention provides the insight that a MSC cultured in the presence of phenanthroline is useful for treating a variety of diseases, in particular diseases related to angiogenesis, the invention further provides the use of phenanthroline or an active derivative thereof for improving the angiogenic properties of MSC and/or supernatant thereof. Also provided is the use of phenanthroline or an active derivative thereof for inducing endothelial differentiation in MSC. It is preferred to improve the angiogenic properties of MSC and/or supernatant thereof, or induce endothelial differentiation in MSC *in vitro.* The invention further provides the use of a cell or a culture supernatant obtainable by a method of the invention for the recruitment of endothelial cells, smooth muscle cells or pericytes to the inoculation site.

In another embodiment, the invention provides the use of a cell and/or supernatant obtainable by a method according to the invention, or phenanthroline or an active derivative thereof for improving the angiogenic properties of medical implants. Such medical implant is for instance an artificial bone or joint structure, such as a hip prosthesis or cartilage, or a skin transplant or a stent. By incorporating a cell and/or supernatant obtainable by a method according to the invention, or phenanthroline or an active derivative thereof in such medical implant, angiogenic properties of such medical implant is improved and vascularisation will be improved after implantation. Consequently the implant is more likely to be accepted by the recipient and incorporated within the surrounding biological structures.

A cell or a (concentrated) supernatant obtainable by a method according to the invention can for instance be incorporated in a scaffold (e.g. calcium phosphate ceramic scaffold, electrospun fibre, 3D printed scaffold) or a coating (e.g. calcium phosphatate coating and derivatives) or be used for tissue regeneration (e.g. incorporated in an (artificial) tendon or ligament to be used in tendon and ligament reconstruction, skin transplants, etc.). In another embodiment, the invention provides a cell and/or supernatant obtainable by a method according to the invention, or phenanthroline or an active derivative thereof for treating diabetes, preferably by improving graft survival of Islets of Langerhans. Langerhans' cells are generally transplanted via portal vein injection, but their survival is generally compromised by lack of vascularisation. For instance, incorporation of islets in a gel containing phenanthroline or an active derivative thereof, or a cell or supernatant obtainable by a method according to the invention improves angiogenesis and therefore increases graft survival and activity.

Now that the invention provides the insight that a cell and/or supernatant cultured in the presence of phenanthroline or an active derivative thereof is useful in the treatment of several conditions, such as ischemia, wound healing and tendon damage, the invention further provides a method for treating a subject, the method comprising: administering to said subject, preferably a human subject, a suitable amount of phenanthroline or an active derivative thereof, or a cell or a supernatant obtainable by a method according to the invention. Such treatment preferably results in less ischemic damage, accelerated wound healing and/or angiogenesis. In a preferred embodiment, said subject is suffering from or is at risk of suffering from ischemia. Preferably, the cell or supernatant is obtainable by a method according to the invention, wherein the method makes use of a huma MSC, more preferably a primary huma MSC, most preferably obtained from the subject to be treated.

In a preferred embodiment, a method for treating a subject in need of angiogenesis is provided, the method comprising administering to said person a suitable amount of phenanthroline or an active derivative thereof, or cells and/or supernatant obtainable by a method according to the invention. A subject in need of angiogenesis is for instance a subject suffering or at risk of suffering from heart disease. It is beneficial that novel arteries and veins are formed that replace arteries and veins that are (partially) obstructed. As described, a method according to the invention preferably results in such novel arteries and veins by inducing angiogenesis in a subject in need thereof.

In yet another preferred embodiment, a method for treating a subject in need of wound repair and/or tendon regeneration and/or ligament regeneration is provided, the method comprising administering to said person a suitable amount of phenanthroline or an active derivative thereof, or cells and/or supernatant obtainable by a method according to the invention. Preferably, the step of administering comprises suturing the wound, tendon and/or ligament with a suture and/or a patch comprising a cell or supernatant obtainable by a method according to the invention, and/or comprising phenanthroline or an active derivative thereof. Such method preferably results in improved vascularisation of the wound edges, tendon and/or ligament, which subsequently leads to improved healing of the wound, ligament and/or tendon.

In (chronic and/or ischemic) wound healing, a cell and/or a supernatant obtainable by a method according to the invention is especially useful. Such cell can for instance be applied directly into the wound, but it is also possible to cover the wound with a wound dressing comprising such cell. Of course, a supernatant obtainable by a method according to the invention can also be incorporated in the wound dressing. It is also possible to provide a wound dressing comprising phenanthroline or an active derivative thereof. Without being bound to theory, it is believed that such wound dressing, when placed on the wound, attracts MSC from other sites of the body, for instance from the bone marrow, to the wound. Near the wound, the MSC come into contact with phenanthroline or the active derivative thereof and as a result will improve their angiogenic property. Such wound dressing comprising phenanthroline or an active derivative thereof thus improves wound healing. Of course, it is also possible to provide the wound dressing with both, MSC and phenanthroline or an active derivative thereof, such that MSC not necessarily first have to be attracted from another site of the body.

It can also attract already differentiated endothelial cells as well as smooth muscle cells or even pericytes. Recruitment of endothelial cells in vivo by a means or method of the invention is important. However, other cell types can be recruited to make a functional blood vessel.

The invention thus provides a wound dressing for accelerating the healing process of a wound, the wound dressing comprising a cell and/or a supernatant obtainable by a method according to the invention, and/or phenanthroline or an active derivative thereof.

Further provided is a method for accelerating the healing process of a wound, the method comprising applying to said wound, a wound dressing according to the invention, or a cell and/or supernatant obtainable by a method according to the invention. Such wound dressing will significantly improve wound healing as a result of its ability to improve angiogenic properties within the wound.

Especially in chronic and/or ischemic wounds it is important to induce angiogenesis. In a preferred embodiment, a method for accelerating the healing process of a wound according to the invention is provided, wherein said wound is a chronic wound and/or wherein said wound is an ischemic wound.

In a preferred embodiment the invention provides a cell or a culture supernatant obtainable by a method of the invention for the use in the treatment of damage to the hart following a stroke or other ischemic event. In a referred embodiment said cell or culture supernatant obtainable by a method of the invention is used in cardiac repair following an ischemic event,, preferably stroke.

The invention further provides a medical product, such as a graft, a (micro)pump, an implant or a wound dressing, comprising phenanthroline or an active derivative thereof, a cell, preferably a human cell, obtainable by a method according to the invention, and/or a supernatant obtainable by a method according to the invention. In a preferred embodiment, a medical product according to the invention is provided, wherein the medical product is a medical implant, such as for instance a prosthesis, coated with phenanthroline or an active derivative thereof, or a cell and/or supernatant obtainable by a method according to the invention.

In another preferred embodiment, a medical product according to the invention is provided, wherein the medical product is a suture or a patch comprising phenanthroline or an active derivative thereof, or a cell and/or supernatant obtainable by a method according to the invention. The use of such suture or patch has the advantage that the edges of the wound are in close contact with phenanthroline or the active derivative, the cell or with factors, present in the supernatant, secreted by such cell. This preferably leads to increased vascularisation of the wound edges and faster healing of the wound.

In a further preferred embodiment, a medical product according to the invention is provided wherein the medical product is an implant or graft. The invention thus provides an implant or a graft for transplantation, comprising phenanthroline or an active derivative thereof, or a cell and/or supernatant obtainable by a method according to the invention. It is possible to apply or provide the graft with phenanthroline or the active derivative, or with such cell or (concentrate) of such supernatant (just) before the graft or transplant is transplanted, but it is also possible to culture the graft or implant in the presence of phenanthroline or the active derivative, the supernatant, and/or together with the MSC for a specific period of time, preferably about 48 hours before transplanting the implant or graft into a recipient.

In a preferred embodiment, the graft for transplantation comprises Langerhans' cells. In yet another preferred embodiment, a medical product according to the invention is provided, wherein the medical product is a pump, preferably a micropump, comprising phenanthroline or an active derivative thereof, or a cell and/or supernatant obtainable by a method according to the invention. Suitable pumps, such as for instance osmotic or matrix gel pumps, are known in the art (55). Such pump comprising phenanthroline or an active derivative thereof, or a cell or a (concentrated) supernatant obtainable by a method according to the invention enables continuous administration of said cell or said supernatant to a person in need thereof. Such pump may, for instance, be inserted after surgery in order to provide the compound(s), cells or supernatant in the vicinity of a wound and, consequently, increase wound healing and/or vascularisation.

The invention thus provides a medical product for use in a medical treatment, the medical product comprising phenanthroline or an active derivative thereof, or a cell or a (concentrated) supernatant obtainable by a method according to the invention. Preferably, the medical product comprises a cell or a supernatant obtainable by a method according to the invention, most preferably the medical product comprising a cell obtainable by a method according to the invention. Preferably such medical product is used for replacing a missing biological structure, supporting a damaged biological structure, or enhancing an existing biological structure.

Also provided is a method for replacing a missing biological structure, supporting a damaged biological structure, and/or enhancing an existing biological structure, the method comprising the use of a cell, preferably a human cell, obtainable by a method according to the invention, a supernatant obtainable by a method according to the invention, phenanthroline and/or an active derivative thereof, and/or a medical product according to the invention, to replace, support and/or enhance a biological structure. A biological structure is defined herein as any biological structure, ranging from a thin cell layer, such as for instance cartilage or skin, to a complete organ, such as for instance a heart or (part) of a liver. The biological structure may be present in a living (human) body, but may also be an *in vitro* engineered biological structure in, for instance, a petri-dish.

Because previous screening methods were based mostly on cancer cells and the results thereof can not necessarily be extrapolated to MSC, a novel screening method was developed, based on an immortalized MSC. Using this screening method, we quickly screened a large panel of potential compounds for effects on MSC. Such screening method is useful for screening a large panel of compound for their ability to modulate (i.e. increase or decrease) an angiogenic capacity of a MSC.

The invention further provides a method for screening whether a compound is able to modulate an angiogenic capacity of a MSC, the method comprising
- providing a MSC with a construct comprising an hypoxia response element operably linked to a reporter gene;
- contacting said MSC with the compound; and
- detecting whether said reporter gene is transcribed,
- wherein the transcription of said reporter gene is indicative for whether said compound is able to modulate an angiogenic capacity of a MSC.

In a preferred embodiment, the MSC is a huma MSC, preferably an immortalized MSC. A compound that is able to improve (i.e. increase) an angiogenic capacity of a MSC preferably induces transcription of said reporter gene, whereas a compound which is capable of decreasing an angiogenic capacity of a MSC preferably inhibits transcription of said reporter gene. It is of course also possible to test a combination of compounds for their synergistic or antagonistic properties with regard to the angiogenic properties of a MSC.

A MSC used in a screening method according to the invention can be obtained from the various tissues as described above. Preferably, the MSC used in a screening method according to the invention is a ligament- or tendon-derived MSC. Such cell is especially useful for screening for compounds that are capable of improving ligament or tendon regeneration. Preferably, such MSC is immortalized before being used in a screening method according to the invention.

It is possible to use for instance an (immortalized) MSC and transfect it with a HRE-luciferase construct as described in Said et al (56) or with a 5HRE-ODD-luc construct described in Saha et al (57). Detection of transcription of the reporter gene is then easily achieved by measuring luciferase activity (luminescence) of the cells after contacting said MSC with the compound. A compound which is able to improve angiogenic capacity of a MSC, preferably induces transcription of the reporter gene through the hypoxia responsive element. As a consequence, reporter gene expression is increased, which can be detected, in case of luciferase, by measuring an increase in luminescence in these cells. Vice versa, decreased luminescence is indicative for a compound able to inhibit angiogenesis, which compound may subsequently be used for anti-angiogenic therapy, for instance in anti-tumor therapy.

The invention is further explained and exemplified by the following non-limiting examples.

### Figure legends

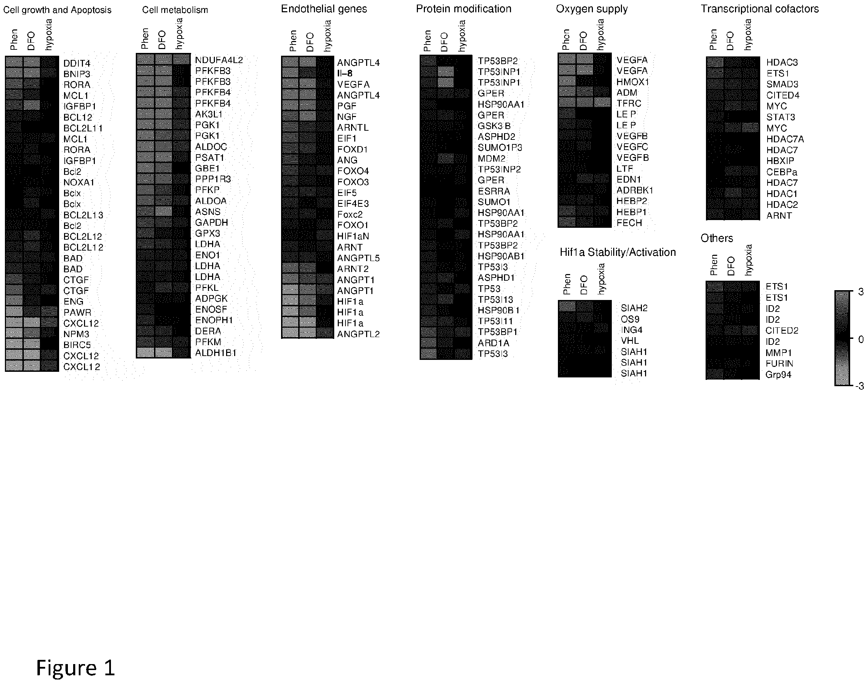

**Figure 1****. Phenanthroline induces strong expression of HRE-containing genes, compared to DFO and hypoxia culture.** Primary MSCs were cultured in the presence of 150 µM DFO, 200 µM PHE or in an hypoxia chamber (2% O₂). After 2 days, whole genome expression analysis was performed and the expression of known HRE-containing genes was examined. Of the three culture methods, most genes were strongest induced using PHE. Heatmaps show the relative expression of denoted genes compared to cells in basic medium, with all genes statistically significant regulated compared to cells in basic medium (P<0.05). The relative expression data are also depicted in table Table 2.

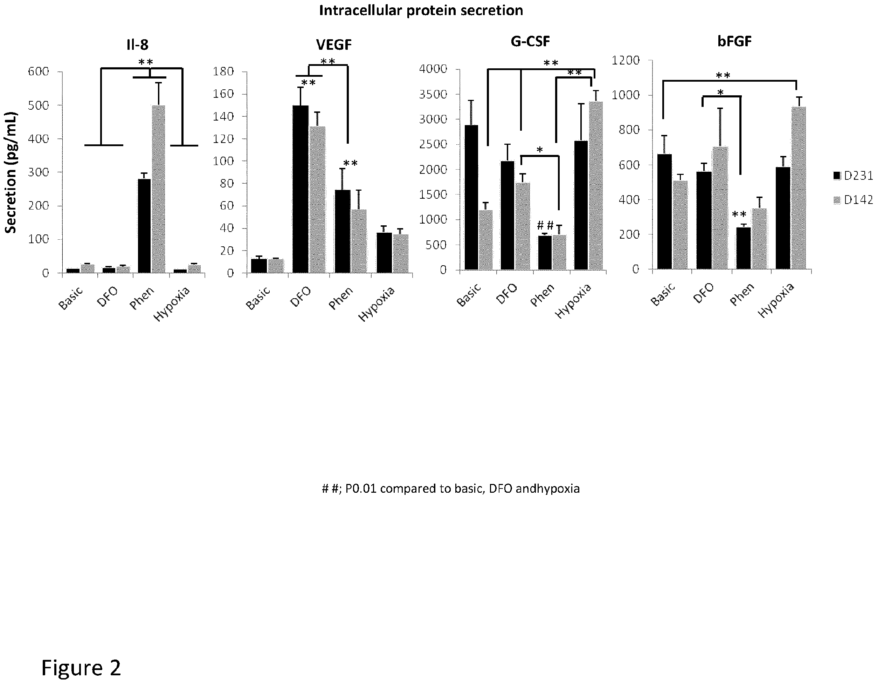

**Figure 2****. Phenanthroline induces secretion of IL-8, but DFO induces higher secretion of VEGF.** Primary MSCs were cultured in the presence of 150 µM DFO, 200 µM PHE or in a hypoxia chamber (2% O₂) for 2 days, after which cells were lysed. Protein concentrations of IL-8, VEGF, bFGF and G-CSF were determined in cell lysates and as shown, only phenanthroline induced secretion of high levels of Il-8, which were not affected by DFO or hypoxia culture. In contrast, VEGF secretion was increased by all three culture methods, but highest by DFO. D231 and D 142; donor number as specified in the laboratory database, (*) denotes P<0.05, (**) denotes P<0.01, (##) denotes P<0.01 compared to basic, DFO and hypoxia (one-way Anova and Tukey's test).

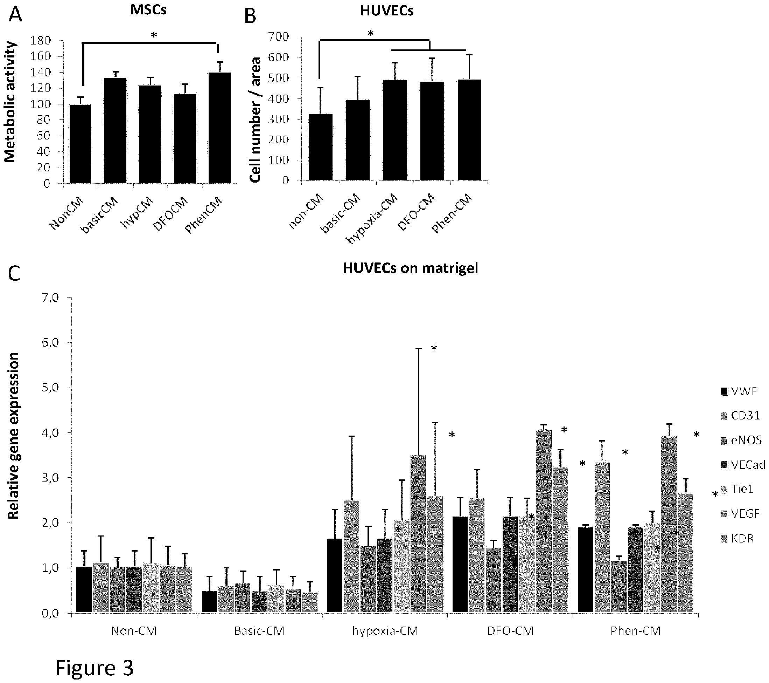

**Figure 3****. Conditioned medium enhances proliferation and sprouting.** Conditioned medium was prepared by culturing cells in the presence of 150 µM DFO, 200 µM PHE or in a hypoxia chamber (2% O₂), after which the medium was changed and cells were kept in culture for 2 more days. As a control non-treated cells were incubated with medium. Conditioned medium was used to culture HUVECs and MSCs and proliferation was determined by counting the cell number or measuring metabolic activity, respectively. **a**, proliferation of MSCs was significantly increased by PHE-CM, but not by other types of CM, although there was no significant difference between PHE-CM and other CM. (*) denotes P<0.05, (One-way Anova and Tukey's test). **b**, in contrast, proliferation of HUVECs was significantly increased by DFO-, PHE- and hypoxia-CM. **c**, gene expression analysis of sprouted HUVECs on matrigel showed that expression of endothelial genes was increased by DFO-CM, PHE-CM and hypoxia-CM, compared to basic- and non-CM. VWF; von Willebrand factor, eNOS; endothelial nitric oxide synthase, VECad; VE cadhering, VEGF; vascular endothelial growth factor, Tie 1; tyrosine kinase receptor, KDR; kinase insert domain receptor, (*) denotes P<0.05 compared to basic-CM (One-way Anova and Tukey's test).

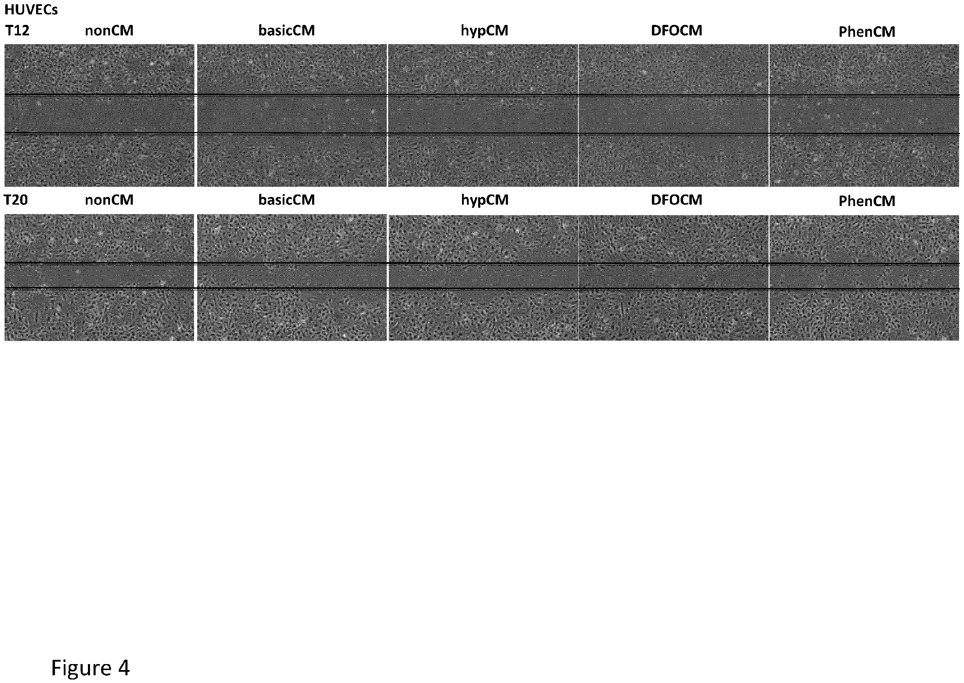

**Figure 4****. Cell migration is enhanced by conditioned medium.** Conditioned medium was prepared by culturing cells in the presence of 150 µM DFO, 200 µM PHE or in a hypoxia chamber (2% O₂), after which the medium was changed and cells were kept in culture for 2 more days. As a control non-treated cells were incubated with medium. Conditioned medium was then used to culture HUVECs and MSCs for a scratch wound healing assay. Migration of cells was increased in conditioned medium, but no differences between these types of conditioned medium were observed, although migration of HUVECs in PHE-CM seemed to be slightly enhanced.
**Figure 5****. In vivo capillary ingrowth in matrigel containing DFO or PHE.** Matrigel plugs (0.5 ml) containing either PBS , DFO (150 uM) or PHE (200 uM) were injected subcutaneously and analyzed for capillary ingrowth after 7 days. **a**, vessel ingrowth was scored in a categorical way and expressed as mean ± SEM per group (n=7) in arbitrary units (AU). **b**, the endothelial nature of the ingrowing cells structures is confirmed by CD31 staining. **c**, in two plugs in the PHE treated group extraordinary ingrowth of capillary like structures throughout the total plug could be observed, demonstrated a clear lumen surrounded by CD31 positive cells.

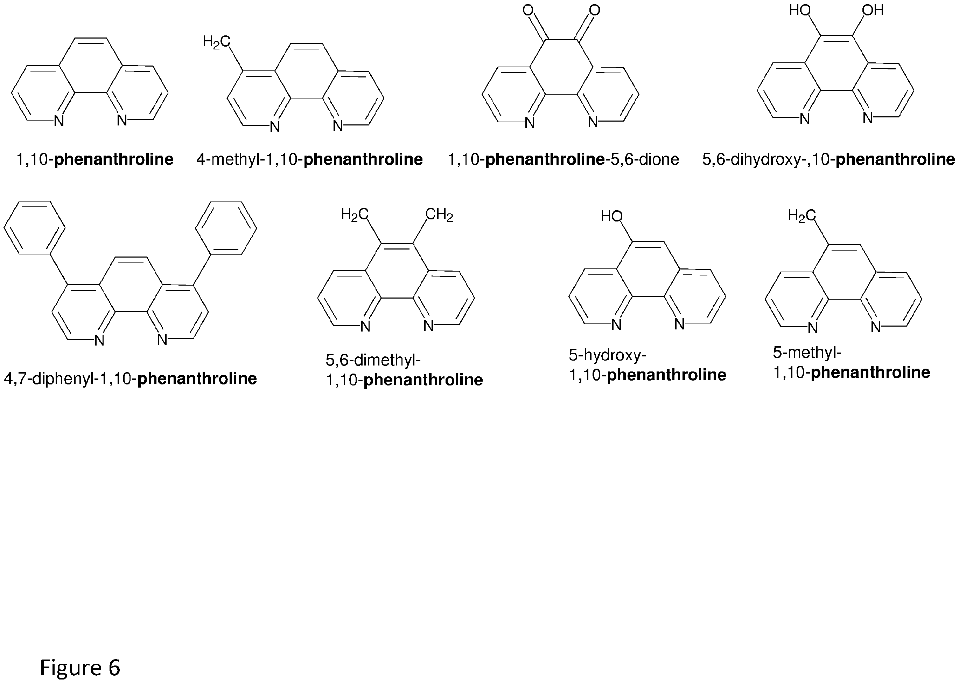

**Figure 6****. Chemical formula of 1,10-phenanthroline and chemical formulas of examples of substituted derivatives.**

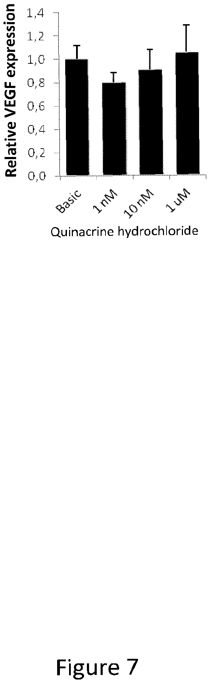

**Figure 7****. Quinacrine hydrochloride does not affect VEGF gene expression.** Primary MSCs were cultured in the presence of quinacrine hydrochloride (1 nM, 10 nM and 1 uM) for 2 days, after which VEGF gene expression was determined by qPCR. None of these concentrations increased VEGF expression.

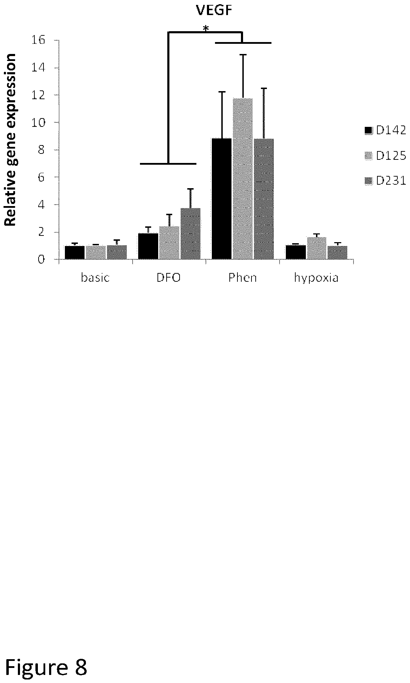

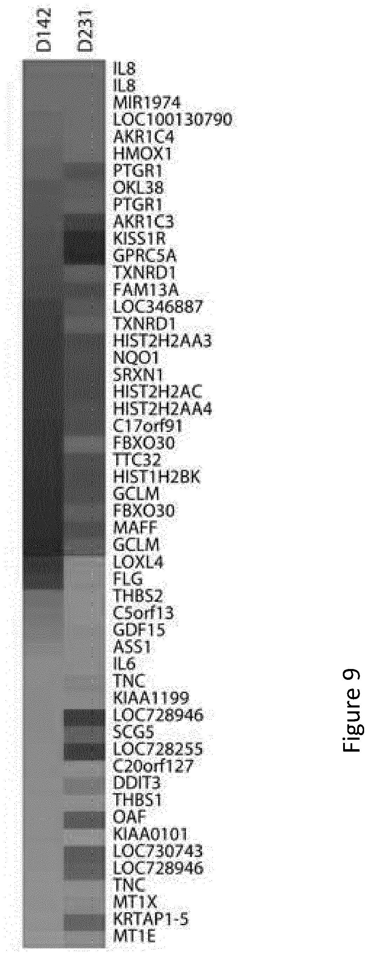

**Figure 8****. Expression of HRE-responsive genes is stably enhanced by PHE.** Primary MSCs were cultured in the presence of DFO, PHE or in an hypoxia chamber for 2 days. After refreshing the medium and 2 more days of culture in basic medium, expression of Il-8, VEGF, KDR and Foxc2 was examined. Only after treatment with PHE expression of these genes was still significantly increased, whereas in DFO and hypoxia cultures expression levels had returned to basal levels.

### Examples

### Materials and methods

### Cell culture

Bone marrow aspirates (5-15mL) were obtained from patients with written informed consent and isolated as previously described (50). Human mesenchymal stromal cells (MSCs) were expanded in proliferation medium consisting of alpha minimal essential medium (alpha-MEM; Gibco, Carlsbad, CA), 10% fetal bovine serum (Lonza, Verviers, Belgium), 0.2 mM ascorbic acid (Sigma Aldrich, St. Louis, MO), 2 mM L-Glutamine (Gibco), 100 U/mL of penicillin and 100 µg/mL of streptomycin (Invitrogen, Carlsbad, CA) and 1 ng/mL of basic fibroblast growth factor (bFGF, Instruchemie, Delfzijl, The Netherlands). Basic medium consisting of proliferation medium without bFGF was used during the experiments. Human umbilical vein endothelial cells (HUVECs) were commercially obtained from Lonza and cultured in Endothelial Growth Medium-2 (EGM-2) with addition of the microvascular bullet kit (MV, all from Clonetics, Lonza), containing hEGF, hydrocortisone, gentamicin, 5% FBS, VEGF, hFGF-B, R3-IGF-1 and ascorbic acid. Cells were kept at 37°C and 5% CO₂. Medium was refreshed three times per week and cells were trypsinised when a confluency of 70-80% was reached.

### Conditioned medium preparation

To prepare conditioned medium (CM), MSCs were cultured until near confluency in proliferation medium. Then, medium was changed to basic medium (basic-CM), basic medium supplemented with 150 µM DFO (DFO-CM) or 200 µM PHE (PHE-CM) or cells were added to a hypoxia chamber (2% O₂). After 2 days of culture, medium was removed and cells were washed with PBS twice, after which serum-free basic medium was added and cells were kept in culture for 2 more days. Then, CM was collected and centrifuged at 900g for 5 minutes to remove cell debris. CM was directly applied to target cells. As a control, serum free medium that had not been in contact with any cells was used (non-CM). For HUVECs, a 7:3 mixture of CM and EGM-2 was used.

### Gene expression analysis

MSCs were seeded in triplicate in 6-well plates at 5000 cells/cm² and allowed to attach for 10-15 hours in proliferation medium. Upon reaching near confluency, cells were treated as described above. After 2 days of treatment and 2 subsequent days of incubation with fresh medium, cells were lysed immediately with TRIzol. RNA was isolated using a Bioke RNA II nucleospin RNA isolation kit (Machery Nagel) and RNA concentrations were measured using an ND100 spectrophotometer (Nanodrop technologies, USA). cDNA was synthesized from 100 ng of RNA, using iScript (BioRad) according to the manufacturer's protocol. For qualitative PCR, a master mix, containing distilled water, forward primer, reverse primer (Sigma Genosys), BSA, and SYBR green I mix (all from Invitrogen) was prepared. Real-time qPCR was performed in a Light-Cycler (Roche). Light-Cycler data was analyzed using the fit points method of Light-Cycler software. The baseline was set at the lower log-linear part above baseline noise and the crossing temperature (Cₜ value) was determined. Ct values were normalized to the 18S housekeeping gene and delta Cₜ (C_{t, control} ― C_{t, sample}) was used to calculate the upregulation in gene expression (53). Primer sequences are listed in table 1.

### Protein expression analysis

MSCs were seeded at 5000 cells/cm² in T25 flasks. Upon reaching near-confluence, medium was changed for basic medium, basic medium with 150 µM DFO or 200 µM PHE or cells were added to a hypoxia chamber (2% O₂). After 2 days, cells were lysed with 250 µL RIPA buffer with addition of protease/phosphatase inhibitors (Roche). Total protein concentrations were determined using a BCA kit (Pierce) and 10 µg of total protein was used to determine concentrations of VEGF, IL-8, basic fibroblast growth factor (bFGF), growth-colony stimulating factor (G-CSF) and epidermal growth factor (EGF) using a Luminex assay (Invitrogen) according to the manufacturer's protocol. Briefly, cells and standards were incubated with fluorescent beads, followed by incubation with a biotinylated detection antibody. After incubation with streptavidin-R-Phycoerythrin and washing, both the fluorescence of the coupled beads and the R-phycoerythrin were measured using a Luminex® FlexMap™.

### Whole genome expression analysis

MSCs were seeded in T25 flasks at 5000 cells/cm² and allowed to attach overnight in proliferation medium. The next day, medium was added with the following conditions; basic medium, basic medium supplemented with 150 uM DFO or basic medium supplemented with 200 µM PHE. After 48 hours, RNA was isolated as described above. From 500 ng of RNA, cRNA was synthesized using the Illumina TotalPrep RNA amplification Kit (Ambion), according to the manufacturer's protocol and the quality of RNA and cRNA was verified on a Bioanalyzer 2100 (Agilent). Microarrays were performed using Illumina HT-12 v4 expression Beadchips, according to the manufacturer's protocol. Briefly, 750 ng of cRNA was hybridized on the array overnight after which the array was washed and blocked. Then, by addition of streptavidin Cy-3 a fluorescent signal was developed. Arrays were scanned on an Illumina iScan reader and raw intensity values were background corrected in GenomeStudio (Illumina). Further data processing and statistical testing were performed with R and Bioconductor statistical software (www.bioconductor.org), using package lumi. Raw intensity values were transformed using variance stabilization and a quantile normalization was performed. A linear modelling approach with empirical Bayesian methods, as implemented in Limma package (54), was applied for differential expression analysis of the resulting probe-level expression values. A gene list, containing only those genes with an absolute 1.5-fold change between different treatments, was uploaded to Ingenuity Pathway Analysis (IPA) software and used for core analysis. Pathways or networks with a p-value of 0.05 were considered statistically significant.

### Proliferation

MSCs and HUVECs were seeded in triplicate in 6-well plates at 3,000 cells/cm² and allowed to attach overnight in denoted culture medium. Then, cells were washed and conditioned medium was added. After 3 days, proliferation of MSCs was determined by measuring the metabolic activity using a 10% (v/v) Alamar Blue (Invitrogen) and for HUVECs, nuclei were stained with DAPI (Sigma Aldrich) and counted.

### Scratch wound healing assay

HUVECs were seeded in triplicate in 6-well plates at 10,000 cells/cm² and allowed to attach for 10 to 15 hours in denoted culture medium. When the cells reached near confluency, a wound was created by scratching the surface with a pipet tip, and the medium was changed to different types of conditioned medium. After 12 and 20 hours pictures were taken to examine migration of cells into the wound.

*HUVEC gene expression profile*6-well plates were coated with 1 mL of a 1:1 mixture of ice-cold Matrigel (Biosciences) and endothelial basic medium (EBM, Lonza). Plates were kept at 37 °C for at least 30 minutes to allow solidification of the Matrigel. Then, HUVECs in passage 2-3 were trypsinized, resuspended in endothelial growth medium-2 (EGM2) (Lonza) and seeded onto the Matrigel at 500,000 cells/well in a volume of 600 µL. Conditioned medium was prepared as described and 1.4 mL of denoted conditioned medium + 0.6 mL of EGM-2 was added to each well. After 24 hours cells were lysed using TRIzol for RNA isolation as described above.

### In vivo murine matrigel plug assay

In vivo angiogenesis analysis was performed using a matrigel plug assay in male C57/BL6 mice (age 8 weeks)(Charles River). Growth factor reduced matrigel (0.5 ml) (BD Biosciences) was injected into the subcutaneous space on the dorsal side of mice on both the left and right flank. Matrigel was mixed at 4 °C with PBS, DFO (150 uM) or PHE (200 uM) (n=7 mice per group). Mice were sacrificed 7 days post-implantation. Matrigel plugs were excised and processed for histological analysis. Paraffin sections (5 um) were stained with Hematoxylin, Phloxine and Saffron (HPS) or anti-CD31 (PECAM, Abcam, Cambridge, UK). Vascular ingrowth was scored in a double blinded fashion and quantified using the following method: every individual section was scored for the degree of ingrowth, using five different categories of ingrowth (1=minimal ingrowth at the border of the plug, 5=capillary ingrowth throughout the total plug), and the mean ingrowth score per group was defined (6 sections per plug, 7 mice per group). The endothelial nature of the infiltrating cells was confirmed by the CD31 staining,

### Statistics

Data was analyzed using one-way ANOVA followed by Tukey's multiple comparison's test (P<0,05). For the analysis of the ingrowth in the matrigel plug statistical analysis was performed using ANOVA and unpaired T test.

### Results

### Phenanthroline induces expression and secretion of angiogenic growth factors

To identify differences between the known hypoxia mimic DFO and PHE, we performed a whole genome expression analysis of MSCs treated for two days and compared this with cells grown under standard hypoxia conditions (2% O₂). Heatmaps in figure 1 show the expression levels of previously identified HRE-containing genes (31), grouped by function. Both DFO and PHE increased expression of genes involved in metabolism, cell growth and survival as well as endothelial genes, but PHE markedly induced higher expression. Expression of various known hypoxia-responsive genes was increased by hypoxia culture, but expression of these genes was markedly higher after treatment with DFO or PHE. Interestingly, interleukin-8 (Il-8) was highly induced by PHE (top gene), whereas DFO and hypoxia did not affect this gene at all.

We then examined the secretion of angiogenic factors at a protein level, including IL-8 and VEGF and, as shown in figure 2, PHE indeed increased the secretion of IL-8 to 300-500 pg/mL, whereas in DFO and hypoxia-treated cells IL-8 levels were comparable to basic (15-25 pg/mL). In contrast, although VEGF secretion was induced by all three treatments, it was higher in DFO-treated cells (140 pg/mL) than in PHE-treated cells (65 pg/mL), even though microarray data showed higher expression of VEGF after treatment with PHE. In contrast, secretion of growth-colony stimulating factor (G-CSF) and basic fibroblast growth factor (bFGF) was reduced by PHE-treatment compared to DFO and hypoxia. To investigate if these secreted growth factors exert trophic effects, conditioned medium (CM) was prepared from DFO-, PHE- or hypoxia-treated MSCs, which was then used to culture fresh HUVECs and MSCs. After 2 days of treatment and 2 more days of incubation with fresh medium, VEGF was still highly expressed in PHE-treated cells, whereas expression levels in DFO- or hypoxia-treated cells were comparable to non-treated cells (Figure 8). However, PHE-CM did not increase proliferation of HUVECs or MSCs, compared to basic- or hypoxia-CM (figure 3A and 3B), although in MSCs, PHE-CM was the only medium to significantly increase proliferation compared to non-CM. To investigate the effect of secreted factors on sprouting of HUVECs, cells were seeded on Matrigel in different types of conditioned medium (CM). As shown in figure 3C, whereas expression of endothelial genes was slightly decreased in basic-CM, DFO-CM and PHE-CM increased expression of these genes and demonstrated similar effects as hypoxia-CM. Similarly, a scratch wound healing assay demonstrated that migration of both HUVECs and MSCs was increased by CM, but no differences were observed between these groups, although for HUVECs, migration seemed slightly increased in DFO- and PHE-CM (Figure 4).

### Phenanthroline enhances in vivo blood vessel formation

Ultimately, we tested the efficacy of DFO and PHE as factors that can stimulate the hypoxia driven angiogenic response in vivo by incorporating DFO (150 uM) or PHE (200 uM) in matrigel plug that are injected subcutaneously in mice. After 7 days a significantly increased capillary ingrowth could be observed in the DFO treated as well as the PHE treated mice (Figure 5). The ingrowth in DFO and PHE did not differ significantly, however it should be noted that in the PHE-treated group two mice showed excessive capillary ingrowth that resulted in the presence of capillary structures throughout the total plug (Figure 5C).

### Discussion

Here we show that treatment of MSCs with such a small molecule, PHE, results in enhanced secretion of VEGF, compared to hypoxia treatment and in addition, enhances vessel formation *in vivo* and results in high protein levels of Il-8, which is involved in proliferation, survival, sprouting and angiogenesis (48). The stronger effects of chemical hypoxia might be the result of the severity of the hypoxia stimulus, which in turn results in a more sustained response. Our data shows that PHE offers a cheap and easy alternative for hypoxia cultures and in addition, PHE can be applied directly in an *in vivo* setup, such as in a matrigel plug. The effects of PHE-conditioned medium on proliferation and sprouting of HUVECs were comparable to those of hypoxia-conditioned medium. Dissolving the compound in water, opposed to the DMSO used here, is likely to reduce the inhibitory effect of PHE on proliferation and improve therapeutic value further. Compared with DFO, PHE treatment had similar effects, but the stronger activation of HIF target genes by PHE shows that this compound is more potent, which is partially reflected in the matrigel plug assay, where PHE enhanced capillary growth in two out of 7 mice.

Whole genome analysis showed that, although DFO and the hypoxia incubator increase expression of several hypoxia target genes, only MSCs exposed to PHE showed a dramatic increase in IL-8 expression. Although described to be upregulated by hypoxia in endothelial cells and fibroblasts (42, 43), IL-8 can also be induced by hypoxia-independent mechanisms. Without being bound by theory, a hypothesis for the increase of IL-8 solely in PHE-treated cells could be that PHE activates another signaling pathway that in turn activates IL-8 transcription. A candidate pathway is the NF-κB pathway, which activation leads to IL-8 expression (44, 45). Indeed, our microarray data shows that expression of RelA - a NF-κB target gene - is higher in the presence of PHE than in hypoxia or DFO-treated cells. Alternatively, increased stability of the heterodimeric complex in the nuclei may explain the stronger activation of HIF target genes, as we also observed a higher expression of P300, a known co-activator of HIF, in PHE-treated cells (21, 46).

Previously, several screens for HIF activation/inhibition have been performed. We used a clonally expanded immortalized MSC cell line reasoning that, the cellular components of the hypoxia pathway will closely resemble those in primary MSCs (39). Surprisingly, of the 12 previously identified hypoxia mimics (28), only phenanthroline or active derivative thereof was able to mimic hypoxia in MSCs. This demonstrates that cell type can be important in regulating hypoxia.

We tested the response to the above-mentioned treatments in MSCs from two different donors and found that the expression profile was highly reproducible.

**Table 1. Primer sequences**

| **Gene** | **Forward primer** | **Reverse primer** |
|---|---|---|
| 18S | CGGCTACCACATCCAAGGAA | GCTGGAATTACCGCGGCT |
| VEGF-A | Commercially obtained from SA biosciences | |

**Table 2. Relative gene expression data**

| Table 2A PHE vs BAS filtered | | | | | |
|---|---|---|---|---|---|
| ProbeID | GeneName | Refseq | Entrez | logFC(D142_PHE - D142_BAS) | adj.P.Val(D142_PHE - D142_BAS) |
| ILMN_1707727 | ANGPTL4 | NM_139314.1 | 51129 | 7,28 | 7,33E-28 |
| ILMN_2184373 | IL8 | NM_000584.2 | 3576 | 6,28 | 1,10E-25 |
| ILMN_1756573 | NDUFA4L2 | NM_020142.3 | 56901 | 5,61 | 1,52E-25 |
| ILMN_1784294 | CPA4 | NM_016352.2 | 51200 | 5,44 | 2,81E-27 |
| ILMN_1666733 | IL8 | NM_000584.2 | 3576 | 5,35 | 1,98E-25 |
| ILMN_2186061 | PFKFB3 | NM_004566.2 | 5209 | 5,23 | 6,16E-26 |
| ILMN_1675947 | MT3 | NM_005954.2 | 4504 | 4,91 | 6,68E-24 |
| ILMN_1765796 | ENO2 | NM_001975.2 | 2026 | 4,72 | 4,25E-23 |
| ILMN_2188862 | GDF15 | NM_004864.1 | 9518 | 4,56 | 9,53E-23 |
| ILMN_1682599 | GPRC5A | NM_003979.3 | 9052 | 4,51 | 2,67E-25 |
| ILMN_1661599 | DDIT4 | NM_019058.2 | 54541 | 4,48 | 1,28E-24 |
| ILMN_1758164 | STC1 | NM_003155.2 | 6781 | 4,35 | 2,08E-24 |
| ILMN_2375879 | VEGFA | NM_003376.4 | 7422 | 4,30 | 7,69E-22 |
| ILMN_1756417 | ANKRD37 | NM_181726.2 | 353322 | 4,25 | 1,37E-24 |
| ILMN_2108735 | EEF1A2 | NM_001958.2 | 1917 | 4,24 | 1,56E-22 |
| ILMN_1653292 | PFKFB4 | NM_004567.2 | 5210 | 4,21 | 2,12E-23 |
| ILMN_1659027 | SLC2A1 | NM_006516.1 | 6513 | 4,08 | 1,41E-23 |
| ILMN_1803882 | VEGFA | NM_001025367.1 | 7422 | 4,07 | 9,09E-23 |
| ILMN_1809931 | NDRG1 | NM_006096.2 | 10397 | 4,06 | 3,22E-23 |
| ILMN_1843198 | | | | 4,02 | 4,25E-23 |
| ILMN_1792356 | DPYSL4 | NM_006426.1 | 10570 | 3,91 | 5,18E-21 |
| ILMN_1680139 | MAFF | NM_012323.2 | 23764 | 3,87 | 8,15E-22 |
| ILMN_1651496 | HIST1H2BD | NM_138720.1 | 3017 | 3,85 | 1,74E-22 |
| ILMN_1744963 | ERO1L | NM_014584.1 | 30001 | 3,84 | 4,67E-23 |
| ILMN_1691884 | STC2 | M_003714.2 | 8614 | 3,81 | 1,61E-22 |
| ILMN_1659047 | HIST2H2AA3 | NM_003516.2 | 8337 | 3,78 | 8,71E-23 |
| ILMN_3242900 | HIST2H2AA4 | NM_001040874.1 | 723790 | 3,75 | 1,09E-20 |
| ILMN_1767556 | C10orf10 | NM_007021.2 | 11067 | 3,71 | 1,56E-22 |
| ILMN_1725139 | CA9 | NM_001216.1 | 768 | 3,69 | 1,68E-22 |
| ILMN_1660847 | PFKFB3 | NM_004566.2 | 5209 | 3,69 | 1,43E-19 |
| ILMN_1772876 | ZNF395 | NM_018660.2 | 55893 | 3,66 | 1,99E-22 |
| ILMN_1659990 | C7orf68 | NM_013332.3 | 29923 | 3,65 | 2,20E-19 |
| ILMN_2144426 | HIST2H2AA3 | NM_003516 2 | 8337 | 3,65 | 2,14E-19 |
| ILMN_1890614 | | | | 3,60 | 1,61E-22 |
| ILMN_1685714 | INHBB | NM_002193 1 | 3625 | 3,60 | 9,57E-22 |
| ILMN_2386444 | ANGPTL4 | NM_0010396671 | 51129 | 3,59 | 2,80E-20 |
| ILMN_2413158 | PODXL | NM_001018111 1 | 5420 | 3,57 | 6,74E-21 |
| ILMN_2361862 | VLDLR | NM_001018056 1 | 7436 | 3,53 | 1,08E-22 |
| ILMN_3308961 | MIR1974 | NR_031738 1 | 1E+08 | 3,51 | 9,04E-17 |
| ILMN_1801077 | PLIN2 | NM_001122 2 | 123 | 3,49 | 1,34E-20 |
| ILMN_1666022 | TNFRSF10D | NM_003840 3 | 8793 | 3,48 | 2,68E-21 |
| ILMN_1724658 | BNIP3 | NM_004052 2 | 664 | 3,47 | 4,02E-23 |
| ILMN_1693334 | P4HA1 | NM_000917 2 | 5033 | 3,46 | 2,01E-19 |
| ILMN_1722532 | JMJD1A | NM_018433 3 | 55818 | 3,45 | 9,20E-19 |
| ILMN_2138765 | PLIN2 | NM_00112 2 | 123 | 3,41 | 1,08E-22 |
| ILMN_1764090 | AK3L1 | NM_203464 1 | 205 | 3,40 | 2,65E-19 |
| ILMN_1768973 | HIST2H2AC | NM_003517 2 | 8338 | 3,39 | 1,33E-19 |
| ILMN_1665510 | ERRFI1 | NM_018948 2 | 54206 | 3,36 | 1.01E-21 |
| ILMN_3181489 | LOC100130790 | XM_001726820 1 | 1E+08 | 3,33 | 1,29E-21 |
| ILMN_2337058 | PORCN | NM_022825 2 | 64840 | 3,30 | 9,88E-21 |
| ILMN_1758623 | HIST1H2BD | M_138720 1 | 3017 | 3,29 | 1,53E-21 |
| ILMN_1809813 | PGF | NM_002632 4 | 5228 | 3,28 | 5,12E-18 |
| ILMN_1752510 | FAM13A | NM_001015045 1 | 10144 | 3,25 | 2,44E-19 |
| ILMN_1704446 | SLC6A10P | NR_003083 2 | 386757 | 3,23 | 4,31E-18 |
| ILMN_1775708 | SLC2A3 | NM_006931 1 | 6515 | 3,23 | 1,79E-20 |
| ILMN_1798249 | AK3L1 | NM_203464 1 | 205 | 3,18 | 1,00E-19 |
| ILMN_2186137 | RRAD | NM_004165 1 | 6236 | 3,18 | 8,49E-19 |
| ILMN_1718866 | C5orf46 | NM_206966 2 | 389336 | 3,15 | 2,64E-17 |
| ILMN_1699772 | RRAGD | NM_021244 3 | 58528 | 3,13 | 3,68E-20 |
| ILMN_1779258 | LOC644774 | XM_927868 1 | 644774 | 3,09 | 7,13E-21 |
| ILMN_1656501 | DUSP5 | NM_004419 3 | 1847 | 3,07 | 2,80E-20 |
| ILMN_1792455 | TMEM158 | NM_015444 2 | 25907 | 3,06 | 1,77E-19 |
| ILMN_1673521 | KISS1R | NM_032551 3 | 84634 | 3,05 | 1,09E-18 |
| ILMN_2322375 | MAFF | NM_012323 2 | 23764 | 3,04 | 7,32E-17 |
| ILMN_1777513 | KCTD11 | NM_001002914 2 | 147040 | 3,02 | 9,05E-18 |
| ILMN_2216852 | PGK1 | NM_000291 2 | 5230 | 2,97 | 8,62E-20 |
| ILMN_1664855 | PPP1R14C | NM_030949 2 | 81706 | 2,96 | 7,35E-20 |
| ILMN_1798360 | CXCR7 | NM_020311 2 | 57007 | 2,96 | 2,20E-19 |
| ILMN_1794017 | SERTAD1 | NM_013376 3 | 29950 | 2,93 | 4,26E-18 |
| ILMN_2150856 | SERPINB2 | NM_002575 1 | 5055 | 2,92 | 6,83E-19 |
| ILMN_1723486 | HK2 | NM_000189 4 | 3099 | 2,90 | 2,39E-18 |
| ILMN_3267451 | GAPDHL6 | XM_001726954 1 | 729403 | 2,90 | 5,90E-19 |
| ILMN_2225537 | PTGR1 | NM_012212 2 | 22949 | 2,90 | 3,44E-19 |
| ILMN_1718961 | BNIP3L | NM_004331 2 | 665 | 2,88 | 1,42E-18 |
| ILMN_1794863 | CAMK2N1 | NM_018584 5 | 55450 | 2,88 | 1,28E-16 |
| ILMN_2308903 | WFDC3 | NM_181522 1 | 140686 | 2,87 | 6,22E-19 |
| ILMN_1751120 | HIST1H4H | NM_003543 3 | 8365 | 2,86 | 9,37E-19 |
| ILMN_1755749 | PGK1 | NM_000291 2 | 5230 | 2,85 | 2,91E-18 |
| ILMN_2401258 | FAM13A | NM_014883 2 | 10144 | 2,84 | 1,23E-18 |
| ILMN_1733110 | RASSF7 | NM_003475 2 | 8045 | 2,83 | 1,99E-18 |
| ILMN_1755974 | ALDOC | NM_005165 | 230 | 2,83 | 2,27E-19 |
| ILMN_1707342 | LRIG1 | NM_015541 2 | 26018 | 2,82 | 2,37E-19 |
| ILMN_1800512 | HMOX1 | NM_002133 1 | 3162 | 2,82 | 2,90E-19 |
| ILMN_1784300 | TUBA4A | NM_006000 1 | 7277 | 2,80 | 1,09E-16 |
| ILMN_1770228 | KRT34 | NM_021013 3 | 3885 | 2,77 | 2,38E-19 |
| ILMN_1683179 | RRAD | NM_004165 1 | 6236 | 2,75 | 1,28E-16 |
| ILMN_1717056 | TXNRD 1 | NM_001093771 1 | 7296 | 2,75 | 1,82E-19 |
| ILMN_3237270 | LOC100133609 | XM_001720815 1 | 1E+08 | 2,75 | 1,14E-16 |
| ILMN_1692938 | PSAT1 | NM_021154 3 | 29968 | 2,74 | 1,24E-19 |
| ILMN_3283772 | LOC644237 | XR_039184 1 | 644237 | 2,73 | 5,88E-18 |
| ILMN_2150851 | SERPINB2 | NM_002575 1 | 5055 | 2,73 | 8,07E-16 |
| ILMN_1671478 | CKB | NM_0018233 | 1152 | 2,73 | 1,66E-17 |
| ILMN_1749892 | EGLN1 | NM_022051 1 | 54583 | 2,72 | 5,54E-20 |
| ILMN_1679727 | CLK1 | NM_0040712 | 1195 | 2,72 | 7,69E-18 |
| ILMN_1795963 | OKL38 | NM_013370 2 | 29948 | 2,72 | 4,16E-16 |
| ILMN_1667791 | PPFIA4 | NM_015053 1 | 8497 | 2,71 | 2,82E-18 |
| ILMN_2128795 | LRIG1 | NM_015541 2 | 26018 | 2,70 | 1,92E-19 |
| ILMN_1720373 | SLC7A5 | NM_003486 5 | 8140 | 2,69 | 3,12E-18 |
| ILMN_1789702 | GBE1 | NM_000158 2 | 2632 | 2,68 | 7,33E-19 |
| ILMN_1774390 | LOC441054 | XM_498987 2 | 441054 | 2,68 | 3,51E-17 |
| ILMN_2227248 | SLAMF9 | NM_033438 1 | 89886 | 2,67 | 4,76E-15 |
| ILMN_1660654 | CDCA2 | NM_152562 2 | 157313 | 2,66 | 9,49E-19 |
| ILMN_1659936 | PPP1R15A | NM_014330 2 | 23645 | 2,65 | 1,65E-19 |
| ILMN_1682717 | IER3 | NM_003897 3 | 8870 | 2,62 | 6,46E-17 |
| ILMN_2338038 | AK3L1 | NM_013410 2 | 205 | 2,61 | 1,40E-18 |
| ILMN_2382942 | CA12 | NM_001218 3 | 771 | 2,61 | 6,22E-19 |
| ILMN_1862909 | | | | 2,60 | 8,29E-17 |
| ILMN_2133187 | POL3S | NM_001039503 2 | 339105 | 2,59 | 1,93E-15 |
| ILMN_2054297 | PTGS2 | NM_000963 1 | 5743 | 2,59 | 2,39E-18 |
| ILMN_1756992 | MUC1 | NM_001044391 1 | 4582 | 2,56 | 3,78E-17 |
| ILMN_1704531 | PTGR1 | NM_012212 2 | 22949 | 2,56 | 3,30E-18 |
| ILMN_2371458 | CXCR7 | NM_001047841 1 | 57007 | 2,54 | 3,01E-16 |
| ILMN_1692785 | KLHL21 | NM_014851 2 | 9903 | 2,54 | 1,29E-17 |
| ILMN_1699265 | TNFRSF10B | NM_0038423 | 8795 | 2,54 | 2,63E-15 |
| ILMN_1703330 | FEM1C | NM_020177 2 | 56929 | 2,53 | 8,04E-19 |
| ILMN_1703123 | AXUD1 | NM_033027 2 | 6465 1 | 2,51 | 1,01E-16 |
| ILMN_1674376 | ANGPTL4 | NM_139314 1 | 51129 | 2,49 | 4,00E-14 |
| ILMN_1708934 | ADM | NM_001124 1 | 133 | 2,48 | 2,04E-17 |
| ILMN_1739942 | FAM117B | NM_173511 2 | 150864 | 2,48 | 4,26E-16 |
| ILMN_2173451 | GPI | NM_000175 2 | 2821 | 2,47 | 9,81E-17 |
| ILMN_2404135 | RIOK3 | NM_003831 2 | 8780 | 2,47 | 4,27E-17 |
| ILMN_1714741 | LOC346887 | XM_943533 1 | 346887 | 2,46 | 5,70E-18 |
| ILMN_1777499 | LOC731007 | XM_001132080 1 | 731007 | 2,46 | 1,84E-17 |
| ILMN_1790778 | PNMA2 | NM_007257 4 | 10687 | 2,46 | 7,49E-16 |
| ILMN_1806349 | SLC6A8 | NM_005629 1 | 6535 | 2,45 | 2,54E-15 |
| ILMN_1749838 | MZF1 | NM_198055 1 | 7593 | 2,45 | 4,11E-16 |
| ILMN_1797372 | C3orf58 | NM_173552 2 | 205428 | 2,45 | 4,19E-17 |
| ILMN_3233930 | LOC390557 | XM_001726973 1 | 390557 | 2,45 | 2,17E-16 |
| ILMN_1758672 | FAM107B | NM_031453 2 | 83641 | 2,44 | 1,94E-17 |
| ILMN_1736670 | PPP1R3C | NM_005398 4 | 5507 | 2,44 | 5,46E-17 |
| ILMN_1694810 | PANX2 | NM_052839 2 | 56666 | 2,42 | 6,46E-17 |
| ILMN_1750912 | STXBP6 | NM_014178 6 | 29091 | 2,42 | 1,15E-17 |
| ILMN_1813314 | HIST1H2BK | NM_080593 1 | 85236 | 2,41 | 2,26E-16 |
| ILMN_1720998 | CA12 | NM_001218 3 | 771 | 2,41 | 4,27E-17 |
| ILMN_1674243 | TFRC | NM_003234 1 | 7037 | 2,40 | 2,90E-18 |
| ILMN_1724700 | RIOK3 | NM_003831 3 | 8780 | 2,40 | 3,19E-18 |
| ILMN_1773407 | C16orf72 | NM_014117 2 | 29035 | 2,40 | 5,78E-17 |
| ILMN_2374865 | ATF3 | NM_001040619 1 | 467 | 2,39 | 8,20E-15 |
| ILMN_1655796 | Mar-03 | XM_001127871 1 | 115123 | 2,37 | 6,13E-17 |
| ILMN_1672350 | JAM2 | NM_021219 2 | 58494 | 2,37 | 3,43E-16 |
| ILMN_2380163 | PTPRF | NM_002840 3 | 5792 | 2,36 | 1,57E-16 |
| ILMN_1775743 | BTG1 | NM_001731 1 | 694 | 2,36 | 4,15E-16 |
| ILMN_1803647 | FAM162A | NM_014367 3 | 26355 | 2,36 | 8,92E-14 |
| ILMN_2132982 | IGFBP5 | NM_000599 2 | 3488 | 2,36 | 1,60E-15 |
| ILMN_1750324 | IGFBP5 | NM_000599 2 | 3488 | 2,34 | 2,44E-16 |
| ILMN_3245418 | FAM180A | NM_205855 2 | 389558 | 2,34 | 4,24E-17 |
| ILMN 2045419 | BNIP3L | NM_004331 2 | 665 | 2,33 | 2,83E-16 |
| ILMN_1668861 | LOC732165 | XM_001134259 1 | 732165 | 2,33 | 6,90E-16 |
| ILMN_3280402 | LOC100132510 | XM_001725190 1 | 1E+08 | 2,32 | 4,67E-13 |
| ILMN_1805737 | PFKP | NM_002627 3 | 5214 | 2,32 | 6,18E-18 |
| ILMN_1804822 | SRXN1 | NM_080725 1 | 140809 | 2,32 | 2,36E-16 |
| ILMN_2324421 | TXNRD1 | NM_182743 1 | 7296 | 2,30 | 4,11E-16 |
| ILMN_2103547 | GOLGA8B | NM_001023567 2 | 440270 | 2,29 | 7,60E-15 |
| ILMN_1815023 | PIM1 | NM_002648 2 | 5292 | 2,29 | 2,51E-16 |
| ILMN_2364022 | SLC16A3 | NM_004207 2 | 9123 | 2,29 | 3,30E-15 |
| ILMN_1775380 | SMOX | NM_175842 1 | 54498 | 2,29 | 8,13E-16 |
| ILMN_1741148 | ALDOA | NM_184043 1 | 226 | 2,28 | 1,28E-16 |
| ILMN_2381697 | P4HA2 | NM_001017974 1 | 8974 | 2,27 | 2,28E-16 |
| ILMN_2371911 | MUC1 | M_001044390 1 | 4582 | 2,27 | 3,97E-15 |
| ILMN_3237448 | BEND5 | NM_024603 2 | 79656 | 2,26 | 6,24E-16 |
| ILMN_3228688 | LOC730415 | XM_001720835 1 | 730415 | 2,25 | 8,25E-17 |
| ILMN_3237511 | C4orf47 | NM_001114357 1 | 441054 | 2,24 | 8,23E-16 |
| ILMN_1726456 | SLC3A2 | NM_001013251 1 | 6520 | 2,24 | 2,80E-16 |
| ILMN_1815121 | PLAGL1 | NM_001080951 1 | 5325 | 2,24 | 8,26E-17 |
| ILMN_1752226 | P2RY11 | NM_002566 4 | 5032 | 2,23 | 9,51E-16 |
| ILMN_1796179 | HIST1H2BK | NM_080593 1 | 85236 | 2,23 | 1,80E-15 |
| ILMN_1676899 | YEATS2 | NM_018023 3 | 55689 | 2,21 | 3,58E-16 |
| ILMN_1699644 | Mar-03 | XM_001127871 1 | 115123 | 2,20 | 1,32E-14 |
| ILMN 3188106 | CYTH2 | NM_017457 4 | 9266 | 2,19 | 4,76E-16 |
| ILMN_1684158 | GPT2 | NM_133443 1 | 84706 | 2,19 | 1,57E-16 |
| ILMN_1751228 | C20orf46 | NM_018354 1 | 55321 | 2,17 | 6,13E-15 |
| ILMN_1676629 | INSIG2 | NM_016133 2 | 51141 | 2,16 | 7,69E-16 |
| ILMN_1671731 | AVPI1 | NM_021732 1 | 60370 | 2,16 | 4,35E-16 |
| ILMN_1738742 | PLAT | NM_000930 2 | 5327 | 2,16 | 2,54E-16 |
| ILMN_1801313 | SIAH2 | NM_005067 5 | 6478 | 2,15 | 4,06E-15 |
| ILMN_1679041 | SLC3A2 | NM_001013251 1 | 6520 | 2,15 | 7,21E-14 |
| ILMN_1748883 | CDKN2D | NM_0794212 | 1032 | 2,14 | 6,70E-16 |
| ILMN_1677511 | PTGS2 | NM_0009631 | 5743 | 2,13 | 6,45E-13 |
| ILMN_2322498 | RORA | NM_0029432 | 6095 | 2,13 | 1,04E-13 |
| ILMN_3305475 | LOC729708 | XM_0017257001 | 729708 | 2,12 | 8,75E-16 |
| ILMN_1764177 | JARID2 | NM_0049732 | 3720 | 2,12 | 6,46E-17 |
| ILMN_2342066 | METRNL | NM_001004431 1 | 284207 | 2,12 | 5,35E-15 |
| ILMN_1694240 | MAP2K1 | NM_002755 2 | 5604 | 2,11 | 2,41E-15 |
| ILMN_1653466 | HES4 | NM_021170 2 | 57801 | 2,11 | 2,65E-14 |
| ILMN_1792689 | HIST1H2AC | NM_003512 3 | 8334 | 2,10 | 6,88E-16 |
| ILMN_1810560 | P8 | NM_012385 1 | 26471 | 2,09 | 4,50E-13 |
| ILMN_2358069 | MAD1L1 | NM_001013836 1 | 8379 | 2,09 | 2,64E-13 |
| ILMN_1684114 | LOC286016 | NR_002187 2 | 286016 | 2,09 | 1,09E-16 |
| ILMN_1689274 | NIPA1 | NM_144599 3 | 123606 | 2,08 | 2,54E-14 |
| ILMN_1662619 | TFPI | NM_006287 4 | 7035 | 2,06 | 1,04E-16 |
| ILMN_1789991 | Mar-04 | NM_020814 1 | 57574 | 2,06 | 5,90E-16 |
| ILMN_3237966 | FBXL11 | NM_012308 1 | 22992 | 2,06 | 6,19E-15 |
| ILMN_1716608 | NGF | NM_002506 2 | 4803 | 2,06 | 2,25E-15 |
| ILMN_1732612 | SHB | NM_003028 2 | 6461 | 2,06 | 2,28E-14 |
| ILMN_1716733 | MYOM2 | NM_003970 1 | 9172 | 2,05 | 1,78E-14 |
| ILMN_2405305 | ARNTL | NM_001030272 1 | 406 | 2,05 | 2,86E-15 |
| ILMN_2367258 | SMOX | NM_175840 1 | 54498 | 2,05 | 1,99E-14 |
| ILMN_1758626 | IDS | NM_000202 3 | 3423 | 2,05 | 2,59E-16 |
| ILMN_1801156 | RLF | NM_012421 2 | 6018 | 2,05 | 1,34E-13 |
| ILMN_1757877 | HCFC1R1 | NM_001002018 1 | 54985 | 2,05 | 4,32E-17 |
| ILMN_1667432 | HYAL3 | NM_003549 2 | 8372 | 2,05 | 3,41E-15 |
| ILMN_1679428 | CHIC2 | NM_012110 2 | 26511 | 2,04 | 1,45E-16 |
| ILMN_3291053 | LOC346085 | XR_019389 1 | 346085 | 2,04 | 1,27E-13 |
| ILMN_2390974 | DNAJB2 | NM_006736 5 | 3300 | 2,04 | 1,28E-16 |
| ILMN_1707124 | TFPI | NM_006287 4 | 7035 | 2,04 | 8,73E-16 |
| ILMN_1801610 | METRNL | XM_941466 2 | 284207 | 2,03 | 3,56E-12 |
| ILMN_1686750 | MGEA5 | NM_012215 2 | 10724 | 2,03 | 4,49E-15 |
| ILMN_1786139 | VKORC1 | NM_024006 4 | 79001 | 2,02 | 1,08E-12 |
| ILMN_1816244 | | | | 2,02 | 1,74E-15 |
| ILMN_1723412 | ASCL2 | NM_005170 2 | 430 | 2,02 | 3,54E-13 |
| ILMN_1658110 | C18orf19 | NM_152352 1 | 125228 | 2,02 | 4,22E-16 |
| ILMN_2374340 | PLAUR | NM_001005376 1 | 5329 | 2,01 | 3,62E-15 |
| ILMN_2174612 | CNOT8 | NM_004779 4 | 9337 | 2,01 | 3,11E-14 |
| ILMN_1827736 | | | | 2,01 | 1,75E-14 |
| ILMN_2408543 | PLAUR | NM_002659 2 | 5329 | 2,01 | 4,47E-15 |
| ILMN_1655930 | ELL2 | NM_012081 4 | 22936 | 2,00 | 6,97E-13 |
| ILMN_2129505 | CYBASC3 | NM_153611 3 | 220002 | -2,00 | 2,85E-15 |
| ILMN_1747016 | CEP55 | NM_018131 3 | 55165 | -2,01 | 8,25E-16 |
| ILMN_1777325 | STAT1 | NM_007315 2 | 6772 | -2,01 | 5,16E-15 |
| ILMN_1666545 | GCNT1 | NM_001097635 1 | 2650 | -2,01 | 8,75E-15 |
| ILMN_2157240 | MNS1 | NM_018365 1 | 55329 | -2,02 | 7,34E-16 |
| ILMN_1657962 | LOC728946 | XM_001128870 1 | 728946 | -2,02 | 2,32E-12 |
| ILMN_1661346 | LOC648210 | XR_018923 1 | 648210 | -2,02 | 1,42E-15 |
| ILMN_1725241 | GSTK1 | NM_015917 1 | 373156 | -2,02 | 1,28E-16 |
| ILMN_3212373 | LOC727803 | XR_039632 1 | 727803 | -2,02 | 2,10E-14 |
| ILMN_1699980 | TSPAN18 | NM_130783 3 | 90139 | -2,03 | 5,59E-14 |
| ILMN_1731966 | LEPR | NM_001003679 1 | 3953 | -2,03 | 1,60E-16 |
| ILMN_1669584 | ILF3 | NM_004516 2 | 3609 | -2,03 | 4,72E-14 |
| ILMN_1708672 | ACAT2 | NM_005891 2 | 39 | -2,03 | 1,04E-14 |
| ILMN_1741200 | RFX5 | NM_001025603 1 | 5993 | -2,04 | 3,45E-14 |
| ILMN_1690105 | STAT1 | NM_007315 2 | 6772 | -2,04 | 3,31E-15 |
| ILMN_1681283 | HIF1A | NM_001530 2 | 3091 | -2,05 | 4,81E-13 |
| ILMN_1802654 | GLT8D2 | NM_031302 3 | 83468 | -2,05 | 1,98E-16 |
| ILMN_2212909 | MELK | NM_014791 2 | 9833 | -2,05 | 5,49E-15 |
| ILMN_1764764 | MUM1 | NM_032853 2 | 84939 | -2,05 | 9,86E-15 |
| ILMN_3305949 | LOC730246 | XR_016080 2 | 730246 | -2,06 | 6,16E-16 |
| ILMN_1668345 | OAF | NM_178507 2 | 220323 | -2,06 | 1,94E-13 |
| ILMN_1684554 | COL16A1 | NM_001856 3 | 1307 | -2,06 | 1,11E-13 |
| ILMN_1794132 | NDUFS8 | NM_002496 1 | 4728 | -2,07 | 1,96E-15 |
| ILMN_1687751 | BAALC | NM_001024372 1 | 79870 | -2,07 | 1,98E-15 |
| ILMN_1769158 | ISOC2 | NM_024710 1 | 79763 | -2,08 | 2,20E-13 |
| ILMN_1709043 | C9orf46 | NM_018465 2 | 55848 | -2,08 | 7,52E-17 |
| ILMN_1729288 | C1QTNF6 | NM_031910 3 | 114904 | -2,08 | 8,25E-16 |
| ILMN_1696601 | VARS | NM_006295 2 | 7407 | -2,08 | 4,76E-16 |
| ILMN_1651958 | MGP | NM_000900 2 | 4256 | -2,09 | 4,22E-16 |
| ILMN_2324002 | CALD1 | NM_033157 2 | 800 | -2,11 | 4,48E-16 |
| ILMN_1713499 | WISP1 | NM_003882 2 | 8840 | -2,11 | 6,51E-16 |
| ILMN_1675062 | MYL9 | NM_006097 3 | 10398 | -2,11 | 2,40E-15 |
| ILMN_1667692 | PTGIS | NM_000961 3 | 5740 | -2,11 | 3,53E-14 |
| ILMN_2332553 | MSRB3 | NM_198080 2 | 253827 | -2,12 | 5,02E-16 |
| ILMN_1815626 | DHCR7 | NM_001360 1 | 1717 | -2,12 | 7,45E-15 |
| ILMN_1810628 | KIAA0367 | NM_015225 1 | 23273 | -2,12 | 2,23E-16 |
| ILMN_1696537 | DDIT4L | NM_145244 2 | 115265 | -2,12 | 1,35E-13 |
| ILMN_1712684 | FAM20C | NM_020223 2 | 56975 | -2,12 | 2,78E-14 |
| ILMN_1716552 | ENAH | NM_018212 4 | 55740 | -2,13 | 4,35E-16 |
| ILMN_1726589 | CD248 | NM_020404 2 | 57124 | -2,13 | 4,94E-16 |
| ILMN_1806037 | TK1 | NM_003258 2 | 7083 | -2,14 | 1,43E-11 |
| ILMN_2380771 | AKR1A1 | NM_153326 1 | 10327 | -2,14 | 9,40E-18 |
| ILMN_1723912 | IFI44L | NM_006820 1 | 10964 | -2,15 | 3,35E-15 |
| ILMN_1761941 | C4orf18 | NM_016613 5 | 51313 | -2,15 | 4,78E-15 |
| ILMN_1708486 | CNN2 | NM_004368 2 | 1265 | -2,15 | 1,15E-14 |
| ILMN_1714730 | UBE2C | NM_181803 1 | 11065 | -2,16 | 4,76E-16 |
| ILMN_1815859 | ERCC2 | NM_000400 2 | 2068 | -2,17 | 4,72E-14 |
| ILMN_1738552 | SLC1A3 | NM_004172 3 | 6507 | -2,18 | 1,81E-15 |
| ILMN_1811909 | LOC402221 | XM_938988 1 | 402221 | -2,18 | 1,40E-16 |
| ILMN_1706498 | DSE | NM_013352 2 | 29940 | -2,19 | 7,33E-17 |
| ILMN_2173611 | MT1E | NM_175617 3 | 4493 | -2,20 | 7,30E-16 |
| ILMN_1797728 | HMGCS1 | NM_002130 6 | 3157 | -2,20 | 1,26E-13 |
| ILMN_1814998 | FKSG30 | NM_001017421 1 | 440915 | -2,20 | 3,22E-16 |
| ILMN_2301083 | UBE2C | NM_181800 1 | 11065 | -2,20 | 4,47E-16 |
| ILMN_1729117 | COL5A2 | NM_000393 3 | 1290 | -2,21 | 2,38E-16 |
| ILMN_1728047 | AKR1A1 | NM_006066 2 | 10327 | -2,21 | 1,70E-17 |
| ILMN_2053415 | LDLR | NM_000527 2 | 3949 | -2,22 | 4,17E-16 |
| ILMN_1800091 | RARRES1 | NM_206963 1 | 5918 | -2,22 | 4,11E-16 |
| ILMN_2062701 | GAS1 | NM_002048 1 | 2619 | -2,22 | 2,40E-16 |
| ILMN_1772644 | EML3 | NM_153265 2 | 256364 | -2,23 | 1,49E-14 |
| ILMN_1678842 | THBS2 | NM_003247 2 | 7058 | -2,24 | 3,88E-17 |
| ILMN_3239771 | DLGAP5 | NM_014750 3 | 9787 | -2,24 | 6,08E-15 |
| ILMN_1690921 | STAT2 | NM_005419 2 | 6773 | -2,24 | 2,41E-17 |
| ILMN_1747195 | PSMB8 | NM_148919 3 | 5696 | -2,24 | 2,82E-15 |
| ILMN_1704154 | TNFRSF19 | NM_148957 2 | 55504 | -2,24 | 6,19E-16 |
| ILMN_2379788 | HIF1A | NM_181054 1 | 3091 | -2,24 | 3,20E-15 |
| ILMN_1790461 | C6orf125 | NM_032340 2 | 84300 | -2,25 | 2,04E-16 |
| ILMN_1786065 | UHRF1 | NM_001048201 1 | 29128 | -2,25 | 1,15E-15 |
| ILMN_1776953 | MYL9 | NM_006097 3 | 10398 | -2,25 | 3,27E-13 |
| ILMN_1796471 | GDF5 | NM_000557 2 | 8200 | -2,25 | 3,62E-15 |
| ILMN_2344283 | FMO3 | NM_001002294 1 | 2328 | -2,27 | 2,76E-15 |
| ILMN_2360710 | TPM1 | NM_001018004 1 | 7168 | -2,27 | 8,93E-17 |
| ILMN_1716687 | TPM1 | NM_001018020 1 | 7168 | -2,28 | 2,60E-16 |
| ILMN_1759598 | DLX5 | NM_005221 5 | 1749 | -2,28 | 1,52E-17 |
| ILMN_1787981 | MFAP2 | NM_017459 1 | 4237 | -2,29 | 9,04E-17 |
| ILMN_2390919 | FBLN2 | NM_001998 2 | 2199 | -2,30 | 3,99E-17 |
| ILMN_1711439 | EMILIN1 | NM_007046 1 | 11117 | -2,30 | 2,48E-13 |
| ILMN_1699651 | IL6 | NM_000600 1 | 3569 | -2,31 | 6,37E-16 |
| ILMN_1854469 | | | | -2,31 | 6,40E-18 |
| ILMN_2390338 | UBE2E3 | NM_006357 2 | 10477 | -2,31 | 1,14E-15 |
| ILMN_1798654 | MCM6 | NM_005915 4 | 4175 | -2,31 | 1,18E-17 |
| ILMN_1790689 | CRISPLD2 | NM_031476 2 | 83716 | -2,32 | 2,00E-16 |
| ILMN_1657796 | STMN1 | NM_203401 1 | 3925 | -2,32 | 1,62E-15 |
| ILMN_1703558 | FHL3 | NM_004468 3 | 2275 | -2,34 | 1,69E-14 |
| ILMN_1812091 | FAM20A | NM_017565 2 | 54757 | -2,34 | 3,43E-16 |
| ILMN_3247132 | LOC730743 | XM_001127014 1 | 730743 | -2,35 | 6,35E-15 |
| ILMN_1685441 | ASAP3 | NM_017707 2 | 55616 | -2,35 | 1,09E-16 |
| ILMN_1657683 | C1orf198 | NM_032800 1 | 84886 | -2,35 | 1,61E-17 |
| ILMN_1701441 | LPAR1 | NM_057159 2 | 1902 | -2,35 | 6,46E-17 |
| ILMN_1739496 | PRRX1 | NM_006902 3 | 5396 | -2,36 | 6,73E-18 |
| ILMN_1666305 | CDKN3 | NM_005192 2 | 1033 | -2,37 | 4,47E-16 |
| ILMN_1741356 | PRICKLE1 | NM_153026 1 | 144165 | -2,37 | 1,49E-16 |
| ILMN_1803825 | CXCL12 | NM_000609 4 | 6387 | -2,38 | 3,81E-15 |
| ILMN_1687301 | VCAN | NM_004385 2 | 1462 | -2,38 | 6,18E-18 |
| ILMN_1800697 | LDB2 | NM_001290 2 | 9079 | -2,38 | 2,41E-17 |
| ILMN_1774602 | FBLN2 | NM_001998 2 | 2199 | -2,40 | 1,42E-16 |
| ILMN_2390299 | PSMB8 | NM_004159 4 | 5696 | -2,40 | 1,67E-14 |
| ILMN_1680738 | C5orf13 | NM_004772 1 | 9315 | -2,41 | 2,07E-17 |
| ILMN_2229877 | PCDH18 | NM_019035 2 | 54510 | -2,41 | 2,08E-16 |
| ILMN_1794501 | HAS3 | NM_005329 2 | 3038 | -2,42 | 2,78E-16 |
| ILMN_1676088 | MSRB3 | NM_198080 2 | 253827 | -2,43 | 2,48E-17 |
| ILMN_1756572 | COQ2 | NM_015697 6 | 27235 | -2,49 | 9,32E-17 |
| ILMN_1785891 | PRKD1 | NM_002742 2 | 5587 | -2,51 | 2,07E-17 |
| ILMN_1728934 | PRC1 | NM_199413 1 | 9055 | -2,53 | 3,96E-18 |
| ILMN_1733094 | STEAP1 | NM_012449 2 | 26872 | -2,54 | 1,18E-17 |
| ILMN_1663390 | CDC20 | NM_001255 2 | 991 | -2,55 | 5,35E-16 |
| ILMN_1669119 | LOC728946 | XM_001128870 1 | 728946 | -2,56 | 2,21E-17 |
| ILMN_1740407 | CHSY3 | NM_175856 4 | 337876 | -2,57 | 9,91E-17 |
| ILMN_1805561 | SLC14A1 | NM_015865 2 | 6563 | -2,57 | 7,55E-18 |
| ILMN_1690125 | PDLIM7 | NM_213636 1 | 9260 | -2,57 | 7,11E-16 |
| ILMN_1744487 | C1QTNF5 | NM_015645 2 | 114902 | -2,59 | 9,47E-18 |
| ILMN_1700268 | QPRT | NM_014298 3 | 23475 | -2,59 | 1,28E-16 |
| ILMN_1667893 | TNS3 | NM_022748 10 | 64759 | -2,61 | 1,07E-17 |
| ILMN_1715508 | NNMT | NM_006169 2 | 4837 | -2,62 | 8,14E-20 |
| ILMN_1786612 | PSME2 | NM_002818 2 | 5721 | -2,64 | 4,12E-18 |
| ILMN_1801939 | CCNB2 | NM_004701 2 | 9133 | -2,66 | 6,59E-16 |
| ILMN_1754969 | LMCD1 | NM_014583 2 | 29995 | -2,67 | 2,57E-20 |
| ILMN_1719392 | FH | NM_000143 2 | 2271 | -2,67 | 4,48E-16 |
| ILMN_2110252 | NPM3 | NM_006993 1 | 10360 | -2,68 | 6,88E-19 |
| ILMN_2349459 | BIRC5 | NM-001168 2 | 332 | -2,71 | 2,96E-17 |
| ILMN_1678493 | CHN1 | NM_001025201 1 | 1123 | -2,73 | 8,62E-20 |
| ILMN_1663575 | MGC87042 | XM_001128032 1 | 256227 | -2,77 | 2,77E-18 |
| ILMN_2148785 | GBP1 | NM_002053 1 | 2633 | -2,77 | 3,09E-17 |
| ILMN_1691364 | STAT1 | NM_139266 1 | 6772 | -2,77 | 1,53E-18 |
| ILMN_1765990 | KCNK2 | NM_001017425 2 | 3776 | -2,82 | 3,12E-18 |
| ILMN_1763260 | HIF1A | NM_001530 2 | 3091 | -2,83 | 4,42E-19 |
| ILMN_1808657 | FMO3 | NM_006894 4 | 2328 | -2,85 | 1,15E-17 |
| ILMN_2234956 | LEPR | NM_001003679 1 | 3953 | -2,85 | 4,08E-18 |
| ILMN_1702363 | SULF1 | M_015170 1 | 23213 | -2,90 | 8,61E-19 |
| ILMN_1790338 | PRRX2 | NM_016307 3 | 51450 | -2,93 | 2,27E-19 |
| ILMN_2388547 | EPSTI1 | NM_033255 2 | 94240 | -2,93 | 3,21E-18 |
| ILMN_1772612 | ANGPTL2 | NM_012098 2 | 23452 | -2,93 | 6,80E-20 |
| ILMN_1725090 | CTHRC1 | NM_138455 2 | 115908 | -2,95 | 1,19E-19 |
| ILMN_2145670 | TNC | NM_002160 1 | 3371 | -2,96 | 1,28E-16 |
| ILMN_1705750 | TGM2 | NM_004613 2 | 7052 | -2,98 | 1,33E-19 |
| ILMN_1765701 | LOC399942 | XM_934471 1 | 399942 | -2,98 | 2,89E-18 |
| ILMN_1686116 | THBS1 | NM_003246 2 | 7057 | -3,00 | 2,94E-20 |
| ILMN_1765557 | OLFML2B | NM_015441 1 | 25903 | -3,04 | 3,54E-19 |
| ILMN_1676663 | TNFRSF11B | NM_002546 3 | 4982 | -3,04 | 5,10E-20 |
| ILMN_1810836 | PDE5A | NM_001083 3 | 8654 | -3,04 | 2,23E-19 |
| ILMN_1671703 | ACTA2 | NM_001613 1 | 59 | -3,04 | 1,49E-19 |
| ILMN_1686097 | TOP2A | NM_001067 2 | 7153 | -3,07 | 1,22E-17 |
| ILMN_1719759 | TNC | NM_002160 2 | 3371 | -3,17 | 2,91E-19 |
| ILMN_1727532 | OLFML3 | NM_020190 2 | 56944 | -3,25 | 5,52E-21 |
| ILMN_1695658 | KIF20A | NM_005733 1 | 10112 | -3,25 | 1,02E-20 |
| ILMN_2376953 | KCNK2 | NM_001017425 2 | 3776 | -3,27 | 1,54E-19 |
| ILMN_2285996 | KIAA0101 | NM_014736 4 | 9768 | -3,27 | 3,14E-19 |
| ILMN_1691476 | MYLK | NM_053032 2 | 4638 | -3,27 | 7,42E-20 |
| ILMN_1770290 | CNN2 | NM_201277 1 | 1265 | -3,30 | 1,64E-22 |
| ILMN_2400935 | TAGLN | NM_003186 3 | 6876 | -3,44 | 1,23E-20 |
| ILMN_1689111 | CXCL12 | NM_000609 4 | 6387 | -3,44 | 5,78E-20 |
| ILMN_1784948 | SPOCD1 | NM_144569 4 | 90853 | -3,46 | 1,08E-22 |
| ILMN_1790529 | LUM | NM_002345 3 | 4060 | -3,46 | 7,69E-22 |
| ILMN_1791447 | CXCL12 | NM_199168 2 | 6387 | -3,48 | 3,19E-20 |
| ILMN_1789507 | COL11A1 | NM_001854 3 | 1301 | -3,53 | 3,22E-20 |
| ILMN_2150894 | ALDH1B1 | NM_000692 3 | 219 | -3,56 | 3,80E-20 |
| ILMN_2117508 | CTHRC1 | NM_138455 2 | 115908 | -3,58 | 1,82E-19 |
| ILMN_1772910 | GAS1 | NM_002048 1 | 2619 | -3,75 | 2,27E-18 |
| ILMN_1689088 | COLEC12 | NM_130386 1 | 81035 | -3,75 | 5,67E-22 |
| ILMN_1656057 | PLAU | NM_002658 2 | 5328 | -3,95 | 1,77E-21 |
| ILMN_1733756 | COL12A1 | NM_080645 2 | 1303 | -3,95 | 9,88E-21 |
| ILMN_1813704 | KIAA1199 | NM_018689 1 | 57214 | -4,06 | 1,56E-22 |
| ILMN_2234697 | BEX1 | NM_018476 3 | 55859 | -4,70 | 1,94E-25 |
| ILMN_1738116 | TMEM119 | NM_181724 1 | 338773 | -4,96 | 1,29E-25 |
| ILMN_2307903 | VCAM1 | NM_001078 2 | 7412 | -5,09 | 4,72E-26 |

| Table 2B DFO versus Bas filtered | | | | | |
|---|---|---|---|---|---|
| ProbeID | GeneName | Refseq | Entrez | logFC(D142_ DFO - D142_BAS) | adj P Val(D142_D FO - D142_BAS) |
| ILMN_2188862 | GDF15 | NM_004864 1 | 9518 | 6,31 | 3,83E-26 |
| ILMN_1707727 | ANGPTL4 | NM_139314 1 | 51129 | 6,19 | 3,83E-26 |
| ILMN_1756573 | NDUFA4L2 | NM_020142 3 | 56901 | 5,66 | 2,14E-25 |
| ILMN_1784294 | CPA4 | NM_016352 2 | 51200 | 5,59 | 2,42E-27 |
| ILMN_1661599 | DDIT4 | NM_019058 2 | 54541 | 4,37 | 5,98E-24 |
| ILMN_1765796 | ENO2 | NM_001975 2 | 2026 | 4,08 | 3,37E-21 |
| ILMN_2186061 | PFKFB3 | NM_004566 2 | 5209 | 4,03 | 1,00E-22 |
| ILMN_1692938 | PSAT1 | NM_021154 3 | 29968 | 3,93 | 3,00E-23 |
| ILMN_1676984 | DDIT3 | NM_004083 4 | 1649 | 3,92 | 2,89E-19 |
| ILMN_1770228 | KRT34 | NM_021013 3 | 3885 | 3,81 | 1,84E-22 |
| ILMN_1758164 | STC1 | NM_003155 2 | 6781 | 3,77 | 1,84E-22 |
| ILMN_1744963 | ERO1L | NM_014584 1 | 30001 | 3,58 | 6,82E-22 |
| ILMN_1809931 | NDRG1 | NM_006096 2 | 10397 | 3,57 | 1,62E-21 |
| ILMN_1691884 | STC2 | NM_003714 2 | 8614 | 3,54 | 2,78E-21 |
| ILMN_1653292 | PFKFB4 | NM_004567 2 | 5210 | 3,53 | 3,37E-21 |
| ILMN_1659027 | SLC2A1 | NM_006516 1 | 6513 | 3,46 | 1,83E-21 |
| ILMN_1890614 | | | | 3,41 | 1,62E-21 |
| ILMN_2375879 | VEGFA | NM_003376 4 | 7422 | 3,40 | 7,41E-19 |
| ILMN_1756417 | ANKRD37 | NM_181726 2 | 353322 | 3,40 | 1,07E-21 |
| ILMN_1724658 | BNIP3 | NM_004052 2 | 664 | 3,37 | 1,84E-22 |
| ILMN_1651496 | HIST1H2BD | NM_138720 1 | 3017 | 3,33 | 1,78E-20 |
| ILMN_1725139 | CA9 | NM_001216 1 | 768 | 3,31 | 6,72E-21 |
| ILMN_1843198 | | | | 3,25 | 1,78E-20 |
| ILMN_1693334 | P4HA1 | NM_000917 2 | 5033 | 3,24 | 2,45E-18 |
| ILMN_2374865 | ATF3 | NM_001040619 1 | 467 | 3,23 | 8,76E-18 |
| ILMN_1720373 | SLC7A5 | NM_003486 5 | 8140 | 3,21 | 7,53E-20 |
| ILMN_2361862 | VLDLR | NM_001018056 1 | 7436 | 3,17 | 3,63E-21 |
| ILMN_1787815 | TRIB3 | NM_021158 3 | 57761 | 3,16 | 3,87E-19 |
| ILMN_1767556 | C10orf10 | NM_007021 2 | 11067 | 3,15 | 2,12E-20 |
| ILMN_1803882 | VEGFA | NM_001025367 1 | 7422 | 3,15 | 1,36E-19 |
| ILMN_3305273 | LOC729779 | XR_019592 2 | 729779 | 3,12 | 4,39E-20 |
| ILMN_2186137 | RRAD | NM_004165 1 | 6236 | 3,12 | 3,17E-18 |
| ILMN_2150851 | SERPINB2 | NM_002575 1 | 5055 | 3,06 | 8,95E-17 |
| ILMN_1792356 | DPYSL4 | NM_006426 1 | 10570 | 3,05 | 6,25E-18 |
| ILMN_1659990 | C7orf68 | NM_013332 3 | 29923 | 3,04 | 4,76E-17 |
| ILMN_2150856 | SERPINB2 | NM_002575 1 | 5055 | 3,04 | 5,71E-19 |
| ILMN_1675947 | MT3 | NM_005954 2 | 4504 | 3,00 | 3,99E-18 |
| ILMN_1664855 | PPP1R14C | NM_030949 2 | 81706 | 2,96 | 1,53E-19 |
| ILMN_1801077 | PLIN2 | NM_001122 2 | 123 | 2,94 | 2,48E-18 |
| ILMN_1796417 | ASNS | NM_133436 1 | 440 | 2,93 | 1,50E-20 |
| ILMN_2413158 | PODXL | NM_001018111 1 | 5420 | 2,92 | 2,48E-18 |
| ILMN_1660067 | LOC728285 | XM_001127070 1 | 728285 | 2,89 | 1,99E-17 |
| ILMN_1810725 | FAM129A | NM_052966 2 | 116496 | 2,88 | 6,71E-18 |
| ILMN_2405521 | MTHFD2 | NM_001040409 1 | 10797 | 2,84 | 2,89E-19 |
| ILMN_1683179 | RRAD | NM_004165 1 | 6236 | 2,83 | 1,24E-16 |
| ILMN_1704446 | SLC6A10P | NR_003083 2 | 386757 | 2,82 | 2,91E-16 |
| ILMN_1773337 | DKK1 | NM_012242 2 | 22943 | 2,79 | 2,73E-18 |
| ILMN_1674706 | MTHFD2 | NM_006636 3 | 10797 | 2,79 | 8,64E-17 |
| ILMN_1787212 | CDKN1A | NM_078467 1 | 1026 | 2,78 | 2,98E-17 |
| ILMN_1772876 | ZNF395 | NM_018660 2 | 55893 | 2,75 | 7,85E-19 |
| ILMN_2216852 | PGK1 | NM_000291 2 | 5230 | 2,75 | 1,24E-18 |
| ILMN_1764090 | AK3L1 | NM_203464 1 | 205 | 2,74 | 1,29E-16 |
| ILMN_1699265 | TNFRSF10B | NM_003842 3 | 8795 | 2,74 | 7,76E-16 |
| ILMN_2138765 | PLIN2 | NM_001122 2 | 123 | 2,74 | 6,75E-20 |
| ILMN_1716608 | NGF | NM_002506 2 | 4803 | 2,73 | 4,01E-18 |
| ILMN_2108735 | EEF1A2 | NM_001958 2 | 1917 | 2,72 | 2,98E-17 |
| ILMN_1750324 | IGFBP5 | NM_000599 2 | 3488 | 2,71 | 1,16E-17 |
| ILMN_1658448 | KRTAP2-1 | XM_926554 2 | 81872 | 2,71 | 5,98E-16 |
| ILMN_1667966 | C1orf24 | NM_052966 1 | 116496 | 2,70 | 2,84E-17 |
| ILMN_2337058 | PORCN | NM_022825 2 | 64840 | 2,69 | 3,64E-18 |
| ILMN_1798249 | AK3L1 | NM_203464 1 | 205 | 2,66 | 1,87E-17 |
| ILMN_1714108 | TP53INP1 | NM_033285 2 | 94241 | 2,66 | 4,41E-17 |
| ILMN_1794863 | CAMK2N1 | NM_018584 5 | 55450 | 2,65 | 2,11E-15 |
| ILMN_2386444 | ANGPTL4 | NM_001039667 1 | 51129 | 2,64 | 1,24E-16 |
| ILMN_1694075 | GADD45A | NM_001924 2 | 1647 | 2,63 | 5,15E-18 |
| ILMN_2214197 | TP53INP1 | NM_033285 2 | 94241 | 2,62 | 1,26E-15 |
| ILMN_3237448 | BEND5 | NM_024603 2 | 79656 | 2,61 | 3,11E-17 |
| ILMN_1779258 | LOC644774 | XM_927868 1 | 644774 | 2,61 | 1,13E-18 |
| ILMN_2052208 | GADD45A | NM_001924 2 | 1647 | 2,60 | 1,87E-17 |
| ILMN_3248701 | LOC644350 | XM_927511 3 | 644350 | 2,60 | 2,64E-15 |
| ILMN_1718866 | C5orf46 | NM_206966 2 | 389336 | 2,59 | 7,05E-15 |
| ILMN_1792455 | TMEM158 | NM_015444 2 | 25907 | 2,58 | 2,84E-17 |
| ILMN_1770085 | BTG2 | NM_006763 2 | 7832 | 2,57 | 1,07E-17 |
| ILMN_1809813 | PGF | NM_002632 4 | 5228 | 2,55 | 6,02E-15 |
| ILMN_1665510 | ERRFI1 | NM_018948 2 | 54206 | 2,53 | 3,47E-18 |
| ILMN_2132982 | IGFBP5 | NM_000599 2 | 3488 | 2,53 | 5,68E-16 |
| ILMN_1775708 | SLC2A3 | NM_006931 1 | 6515 | 2,53 | 1,87E-17 |
| ILMN_1699644 | Mar-03 | XM_001127871 1 | 115123 | 2,52 | 8,86E-16 |
| ILMN_1892403 | SNORD13 | NR_003041 1 | 692084 | 2,51 | 1,45E-14 |
| ILMN_1803647 | FAM162A | NM_014367 3 | 26355 | 2,50 | 4,37E-14 |
| ILMN_3237270 | LOC100133609 | XM_001720815 1 | 1E+08 | 2,49 | 2,64E-15 |
| ILMN_1660847 | PFKFB3 | NM_004566 2 | 5209 | 2,48 | 6,31E-15 |
| ILMN_1680139 | MAFF | NM_012323 2 | 23764 | 2,48 | 1,58E-16 |
| ILMN_3248758 | LOC728934 | XM_001128836 2 | 728934 | 2,47 | 3,31E-15 |
| ILMN_1655796 | Mar-03 | XM_001127871 1 | 115123 | 2,46 | 4,43E-17 |
| ILMN_2347068 | MKNK2 | NM_017572 2 | 2872 | 2,45 | 2,57E-15 |
| ILMN_2387385 | IGFBP1 | NM_001013029 1 | 3484 | 2,45 | 7,38E-18 |
| ILMN_1699772 | RRAGD | NM_021244 3 | 58528 | 2,43 | 4,57E-17 |
| ILMN_1758623 | HIST1H2BD | NM_138720 1 | 3017 | 2,40 | 1,07E-17 |
| ILMN_1682599 | GPRC5A | NM_003979 3 | 9052 | 2,35 | 1,07E-17 |
| ILMN_1743199 | EGR2 | NM_000399 2 | 1959 | 2,34 | 3,79E-14 |
| ILMN_1755974 | ALDOC | NM_005165 2 | 230 | 2,33 | 6,34E-17 |
| ILMN_1736670 | PPP1R3C | NM_005398 4 | 5507 | 2,33 | 3,28E-16 |
| ILMN_1726456 | SLC3A2 | NM_001013251 1 | 6520 | 2,31 | 2,77E-16 |
| ILMN_1674243 | TFRC | NM_003234 1 | 7037 | 2,31 | 1,70E-17 |
| ILMN_1789702 | GBE1 | NM_000158 2 | 2632 | 2,29 | 8,34E-17 |
| ILMN_2338038 | AK3L1 | NM_0134102 2 | 205 | 2,28 | 8,93E-17 |
| ILMN_1798081 | PTPRF | NM_130440 1 | 5792 | 2,28 | 1,53E-13 |
| ILMN_1749892 | EGLN1 | NM_022051 1 | 54583 | 2,28 | 1,02E-17 |
| ILMN_1679041 | SLC3A2 | NM_001013251 1 | 6520 | 2,26 | 4,09E-14 |
| ILMN_1794017 | SERTAD1 | NM_013376 3 | 29950 | 2,25 | 6,43E-15 |
| ILMN_2054297 | PTGS2 | NM_000963 1 | 5743 | 2,25 | 1,58E-16 |
| ILMN_1718961 | BNIP3L | NM_004331 2 | 665 | 2,24 | 1,73E-15 |
| ILMN 2113490 | NTN4 | NM_021229 3 | 59277 | 2,23 | 7,70E-15 |
| ILMN_1795697 | PTP4A2 | XM_944907 1 | 8073 | 2,22 | 6,02E-15 |
| ILMN_1723486 | HK2 | NM_000189 4 | 3099 | 2,21 | 4,62E-15 |
| ILMN_1758672 | FAM107B | NM_031453 2 | 83641 | 2,18 | 7,10E-16 |
| ILMN_1704537 | PHGDH | NM_006623 2 | 26227 | 2,18 | 6,63E-17 |
| ILMN_1751598 | SESN2 | NM_031459 3 | 83667 | 2,18 | 3,36E-14 |
| ILMN_3283772 | LOC644237 | XR_039184 1 | 644237 | 2,17 | 4,13E-15 |
| ILMN_1761281 | LOC441019 | XM_498969 2 | 441019 | 2,16 | 6,39E-14 |
| ILMN_1684158 | GPT2 | NM_133443 1 | 84706 | 2,16 | 4,22E-16 |
| ILMN_1755749 | PGK1 | NM_000291 2 | 5230 | 2,16 | 6,62E-15 |
| ILMN_1682717 | IER3 | NM_003897 | 8870 | 2,14 | 1,96E-14 |
| ILMN_2121408 | HBEGF | NM_001945 1 | 1839 | 2,13 | 3,56E-16 |
| ILMN_1722532 | JMJD1A | NM_018433 3 | 55818 | 2,13 | 3,26E-13 |
| ILMN_1777513 | KCTD11 | NM_001002914 2 | 147040 | 2,12 | 1,18E-13 |
| ILMN_2103547 | GOLGA8B | NM_001023567 2 | 440270 | 2,12 | 1,04E-13 |
| ILMN_2098446 | PMAIP1 | NM_021127 1 | 5366 | 2,11 | 1,50E-15 |
| ILMN_3242900 | HIST2H2AA4 | NM_001040874 1 | 723790 | 2,11 | 4,12E-14 |
| ILMN_2144426 | HIST2H2AA3 | NM_003516 2 | 8337 | 2,07 | 5,59E-13 |
| ILMN_1738407 | ULBP1 | NM_025218 2 | 80329 | 2,07 | 4,42E-14 |
| ILMN_2322375 | MAFF | NM_012323 2 | 23764 | 2,07 | 1,77E-12 |
| ILMN_1659047 | HIST2H2AA3 | NM_003516 2 | 8337 | 2,07 | 8,02E-16 |
| ILMN_1685714 | INHBB | NM_002193 1 | 3625 | 2,06 | 2,98E-15 |
| ILMN_1670875 | PPM1D | NM_003620 2 | 8493 | 2,06 | 6,09E-13 |
| ILMN_1672350 | JAM2 | NM_021219 2 | 58494 | 2,05 | 2,41E-14 |
| ILMN_1679727 | CLK1 | NM_004071 2 | 1195 | 2,03 | 2,36E-14 |
| ILMN_1738742 | PLAT | NM_000930 2 | 5327 | 2,03 | 2,61E-15 |
| ILMN_1704154 | TNFRSF19 | NM_148957 2 | 55504 | -2,00 | 2,05E-14 |
| ILMN_2167805 | LUM | NM_002345 3 | 4060 | -2,01 | 7,60E-12 |
| ILMN_2301083 | UBE2C | NM_181800 1 | 11065 | -2,01 | 8,26E-15 |
| ILMN_2053415 | LDLR | NM_000527 2 | 3949 | -2,03 | 8,03E-15 |
| ILMN_1810172 | SFRP4 | NM_003014 2 | 6424 | -2,03 | 1,18E-12 |
| ILMN_1756572 | COQ2 | NM_015697 6 | 27235 | -2,03 | 3,02E-14 |
| ILMN_1785891 | PRKD1 | NM_002742 2 | 5587 | -2,05 | 6,65E-15 |
| ILMN_2222008 | KIFC1 | NM_002263 2 | 3833 | -2,05 | 2,86E-11 |
| ILMN_1742981 | TUBA1A | NM_006009 2 | 7846 | -2,06 | 6,31E-15 |
| ILMN_1690170 | CRABP2 | NM_001878 2 | 1382 | -2,06 | 6,27E-15 |
| ILMN_2376953 | KCNK2 | NM_001017425 2 | 3776 | -2,07 | 3,13E-14 |
| ILMN_1765990 | KCNK2 | NM_001017425 2 | 3776 | -2,07 | 1,48E-14 |
| ILMN_2062701 | GAS1 | NM_002048 1 | 2619 | -2,07 | 2,85E-15 |
| ILMN_1810836 | PDE5A | NM_001083 3 | 8654 | -2,08 | 6,53E-15 |
| ILMN_1797728 | HMGCS1 | NM_002130 6 | 3157 | -2,09 | 8,15E-13 |
| ILMN_1812968 | SOX18 | NM_018419 2 | 54345 | -2,09 | 1,13E-13 |
| ILMN_1653711 | FZD2 | NM_001466 2 | 2535 | -2,10 | 8,02E-15 |
| ILMN_1812091 | FAM20A | NM_017565 2 | 54757 | -2,11 | 8,42E-15 |
| ILMN_2200836 | HSPB7 | NM_014424 3 | 27129 | -2,12 | 2,99E-15 |
| ILMN_1725090 | CTHRC1 | NM_138455 2 | 115908 | -2,13 | 8,26E-16 |
| ILMN_1696537 | DDIT4L | NM_145244 2 | 115265 | -2,14 | 2,14E-13 |
| ILMN_2349459 | BIRC5 | NM_001168 2 | 332 | -2,14 | 2,03E-14 |
| ILMN_1733094 | STEAP1 | NM_012449 2 | 26872 | -2,15 | 1,65E-15 |
| ILMN_1715508 | NNMT | NM_006169 2 | 4837 | -2,16 | 2,33E-17 |
| ILMN_1663575 | MGC87042 | XM_001128032 1 | 256227 | -2,17 | 2,45E-15 |
| ILMN_1686097 | TOP2A | NM_001067 2 | 7153 | -2,19 | 1,17E-13 |
| ILMN_1803825 | CXCL12 | NM_000609 4 | 6387 | -2,19 | 6,04E-14 |
| ILMN_1811909 | LOC402221 | XM_938988 1 | 402221 | -2,22 | 1,70E-16 |
| ILMN_1774602 | FBLN2 | NM_001998 2 | 2199 | -2,23 | 1,70E-15 |
| ILMN_1814998 | FKSG30 | NM_001017421 1 | 440915 | -2,25 | 3,69E-16 |
| ILMN_1770290 | CNN2 | NM_201277 1 | 1265 | -2,29 | 4,69E-18 |
| ILMN_2344283 | FMO3 | NM_001002294 1 | 2328 | -2,30 | 4,06E-15 |
| ILMN_1657683 | C1orf198 | NM_032800 1 | 84886 | -2,30 | 5,69E-17 |
| ILMN_1772612 | ANGPTL2 | NM_012098 2 | 23452 | -2,31 | 5,71E-17 |
| ILMN_1747016 | CEP55 | M_018131 3 | 55165 | -2,32 | 4,25E-17 |
| ILMN_2234956 | LEPR | NM_001003679 1 | 3953 | -2,33 | 1,50E-15 |
| ILMN_1786065 | UHRF1 | NM_001048201 1 | 29128 | -2,34 | 8,87E-16 |
| ILMN_2148785 | GBP1 | NM_002053 1 | 2633 | -2,41 | 2,12E-15 |
| ILMN_1702363 | SULF1 | NM_015170 1 | 23213 | -2,42 | 1,70E-16 |
| ILMN_1690125 | PDLIM7 | NM_213636 1 | 9260 | -2,43 | 6,33E-15 |
| ILMN_1700268 | QPRT | NM_014298 3 | 23475 | -2,44 | 1,15E-15 |
| ILMN_2117508 | CTHRC1 | NM_138455 2 | 115908 | -2,46 | 5,25E-15 |
| ILMN_1808657 | FMO3 | NM_006894 4 | 2328 | -2,46 | 9,45E-16 |
| ILMN_1790338 | PRRX2 | NM_016307 3 | 51450 | -2,49 | 3,11E-17 |
| ILMN_1784948 | SPOCD1 | NM_144569 4 | 90853 | -2,53 | 7,09E-19 |
| ILMN_2150894 | ALDH1B1 | NM_000692 3 | 219 | -2,55 | 3,69E-16 |
| ILMN_1789507 | COL11A1 | NM_001854 3 | 1301 | -2,55 | 2,55E-16 |
| ILMN_1733756 | COL12A1 | NM_080645 2 | 1303 | -2,56 | 1,24E-15 |
| ILMN_1791447 | CXCL12 | NM_199168 2 | 6387 | -2,57 | 1,40E-16 |
| ILMN_1671703 | ACTA2 | NM_001613 1 | 59 | -2,61 | 1,43E-17 |
| ILMN_1728934 | PRC1 | NM_199413 1 | 9055 | -2,66 | 2,45E-18 |
| ILMN_1765701 | LOC399942 | XM_934471 1 | 399942 | -2,67 | 9,45E-17 |
| ILMN_1744487 | C1QTNF5 | NM_015645 2 | 114902 | -2,69 | 7,58E-18 |
| ILMN_1656057 | PLAU | NM_002658 2 | 5328 | -2,69 | 6,84E-17 |
| ILMN_1678493 | CHN1 | NM_001025201 1 | 1123 | -2,71 | 2,13E-19 |
| ILMN_1695658 | KIF20A | NM_005733 1 | 10112 | -2,76 | 1,54E-18 |
| ILMN_1727532 | OLFML3 | NM_020190 2 | 56944 | -2,91 | 2,09E-19 |
| ILMN_1689111 | CXCL12 | NM_000609 4 | 6387 | -2,92 | 7,58E-18 |
| ILMN_1772910 | GAS1 | NM_002048 1 | 2619 | -3,12 | 4,83E-16 |
| ILMN_2307903 | VCAM1 | NM_001078 2 | 7412 | -3,16 | 1,12E-20 |
| ILMN_1765557 | OLFML2B | NM_015441 1 | 25903 | -3,16 | 2,90E-19 |
| ILMN_1689088 | COLEC12 | NM_130386 1 | 81035 | -3,20 | 7,53E-20 |
| ILMN_1790529 | LUM | NM_002345 3 | 4060 | -3,27 | 7,20E-21 |
| ILMN_1663390 | CDC20 | NM_001255 2 | 991 | -3,40 | 7,45E-19 |
| ILMN_2234697 | BEX1 | NM_018476 3 | 55859 | -3,45 | 1,32E-21 |
| ILMN_1738116 | TMEM119 | NM_181724 1 | 338773 | -3,83 | 1,84E-22 |

| Table 2C HYP versus Bas filtered | | | | | |
|---|---|---|---|---|---|
| ProbeID | GeneName | Refseq | Entrez | logFC(D142_HYP - D142_BAS) | adj P Val(D142_HYP -D142_BAS) |
| ILMN_1756573 | NDUFA4L2 | NM_020142 3 | 56901 | 2,36 | 1,50E-13 |
| ILMN 2216852 | PGK1 | NM_000291 2 | 5230 | 1,75 | 7,24E-12 |
| ILMN_1717990 | CALD1 | NM_033138 2 | 800 | 1,61 | 4,92E-10 |
| ILMN_1775380 | SMOX | NM_175842 1 | 54498 | 1,52 | 1,06E-09 |
| ILMN_2367258 | SMOX | NM_175840 1 | 54498 | 1,51 | 1,53E-09 |
| ILMN_1850238 | | | | 1,51 | 1,71E-08 |
| ILMN_1706706 | WDR68 | NM_001003725 1 | 10238 | 1,48 | 1,02E-10 |
| ILMN_1799569 | LOC440895 | XM_934629 1 | 440895 | 1,41 | 2,82E-09 |
| ILMN_1755749 | PGK1 | NM_000291 2 | 5230 | 1,36 | 6,05E-09 |
| ILMN_1755974 | ALDOC | NM_005165 2 | 230 | 1,31 | 2,02E-09 |
| ILMN_1779258 | LOC644774 | XM_927868 1 | 644774 | 1,30 | 1,06E-09 |
| ILMN_3305475 | LOC729708 | XM_001725700 1 | 729708 | 1,29 | 5,26E-09 |
| ILMN_1695706 | H3F3B | NM_005324 3 | 3021 | 1,28 | 1,36E-09 |
| ILMN_1668861 | LOC732165 | XM_001134259 1 | 732165 | 1,24 | 5,32E-08 |
| ILMN_1807074 | MIF | NM_002415 1 | 4282 | 1,23 | 2,82E-09 |
| ILMN_2189675 | HRCT1 | NM_001039792 1 | 646962 | 1,23 | 1,51E-07 |
| ILMN_1691104 | PGAM4 | NM_001029891 2 | 441531 | 1,21 | 1,06E-07 |
| ILMN_3277209 | LOC285943 | XR_040116 1 | 285943 | 1,21 | 1,11E-07 |
| ILMN_1684114 | LOC286016 | NR_002187 2 | 286016 | 1,21 | 2,82E-09 |
| ILMN_1792495 | AHNAK | NM_001620 1 | 79026 | 1,19 | 5,94E-08 |
| ILMN_1886487 | | | | 1,19 | 2,49E-08 |
| ILMN_1752159 | AHNAK | NM_024060 2 | 79026 | 1,18 | 4,28E-09 |
| ILMN_1728059 | LOC645236 | XM_928275 1 | 645236 | 1,18 | 1,31E-07 |
| ILMN_2338038 | AK3L1 | NM_013410 2 | 205 | 1,17 | 1,31E-08 |
| ILMN_1661366 | PGAM1 | NM_002629 2 | 5223 | 1,17 | 3,60E-07 |
| ILMN_3267451 | GAPDHL6 | XM_001726954 1 | 729403 | 1,16 | 5,94E-08 |
| ILMN_1790741 | RNF126 | NM_194460 1 | 55658 | 1,16 | 1,78E-07 |
| ILMN_1660654 | CDCA2 | NM_152562 2 | 157313 | 1,13 | 2,68E-08 |
| ILMN_2366719 | NSFL1C | NM_182483 1 | 55968 | 1,12 | 5,10E-07 |
| ILMN_1674661 | CIRBP | NM_001280 1 | 1153 | 1,12 | 8,21E-09 |
| ILMN_2173451 | GPI | NM_000175 2 | 2821 | 1,11 | 2,33E-07 |
| ILMN_3283772 | LOC644237 | XR_039184 1 | 644237 | 1,08 | 3,02E-07 |
| ILMN_1663042 | SDC4 | NM_002999 2 | 6385 | 1,08 | 1,12E-06 |
| ILMN_1707627 | TPI1 | NM_000365 4 | 7167 | 1,08 | 3,47E-07 |
| ILMN_1791002 | SKP2 | NM_005983 2 | 6502 | 1,08 | 2,34E-05 |
| ILMN_1747160 | SYT15 | NM_181519 2 | 83849 | 1,05 | 1,02E-06 |
| ILMN_1735955 | LOC644033 | XM_927280 1 | 644033 | 1,05 | 1,74E-07 |
| ILMN_1764090 | AK3L1 | NM_203464 1 | 205 | 1,04 | 3,06E-06 |
| ILMN_2112417 | PGAM1 | NM_0026292 2 | 5223 | 1,04 | 1,98E-06 |
| ILMN_1682953 | PGAM4 | NM_001029891 2 | 441531 | 1,03 | 1,06E-07 |
| ILMN_1790562 | EYA3 | NM_001990 2 | 2140 | 1,03 | 8,00E-07 |
| ILMN_1793543 | C1orf51 | NM_144697 2 | 148523 | 1,03 | 9,09E-07 |
| ILMN_1669667 | PDLIM2 | NM_198042 2 | 64236 | 1,03 | 2,06E-06 |
| ILMN_1659703 | WWP2 | NM_199423 1 | 11060 | 1,03 | 4,40E-07 |
| ILMN_1772876 | ZNF395 | NM_018660 2 | 55893 | 1,02 | 1,33E-07 |
| ILMN_2143155 | KIF11 | NM_004523 2 | 3832 | 1,02 | 9,87E-08 |
| ILMN_1724658 | BNIP3 | NM_004052 2 | 664 | 1,01 | 1,70E-08 |
| ILMN_3301740 | LOC729887 | XR_040891 1 | 729887 | 1,01 | 1,89E-05 |
| ILMN_1693334 | P4HA1 | NM_000917 2 | 5033 | 1,01 | 6,01E-06 |
| ILMN_3305938 | SGK1 | NM_005627 3 | 6446 | -1,01 | 6,85E-08 |
| ILMN_3240314 | SCXA | NM_001008271 1 | 1E+08 | -1,02 | 6,50E-06 |
| ILMN_1685854 | C5orf53 | NM_001007189 1 | 492311 | -1,03 | 9,30E-08 |
| ILMN_1789507 | COL11A1 | NM_001854 3 | 1301 | -1,03 | 1,82E-06 |
| ILMN_1754969 | LMCD1 | NM_014583 2 | 29995 | -1,03 | 9,73E-09 |
| ILMN_1664776 | EFR3A | NM_015137 3 | 23167 | -1,03 | 1,03E-07 |
| ILMN_1679476 | GART | NM_000819 3 | 2618 | -1,04 | 2,32E-07 |
| ILMN_1667825 | MLKL | NM_152649 1 | 197259 | -1,04 | 5,38E-06 |
| ILMN_1805561 | SLC14A1 | NM_015865 2 | 6563 | -1,04 | 2,33E-07 |
| ILMN_1663631 | BANP | NM_079837 2 | 54971 | -1,04 | 5,72E-08 |
| ILMN_2397750 | IVNS1ABP | NM_006469 4 | 10625 | -1,04 | 7,83E-07 |
| ILMN_2330570 | LEPR | NM_001003680 1 | 3953 | -1,05 | 1,16E-07 |
| ILMN_1797684 | PDCD2 | NM_002598 2 | 5134 | -1,05 | 2,88E-07 |
| ILMN_2300186 | DYNLL1 | NM_001037494 1 | 8655 | -1,06 | 1,18E-06 |
| ILMN_1696537 | DDIT4L | NM_145244 2 | 115265 | -1,06 | 5,38E-06 |
| ILMN_1702487 | SGK | NM_005627 2 | 6446 | -1,06 | 5,72E-08 |
| ILMN_1740426 | RASD1 | NM_016084 3 | 51655 | -1,07 | 6,09E-07 |
| ILMN_1795227 | DNCL1 | NM_003746 1 | 8655 | -1,07 | 1,13E-07 |
| ILMN_2103591 | MORC2 | NM_014941 1 | 22880 | -1,07 | 1,11E-06 |
| ILMN_2269564 | ARID4B | NM_016374 5 | 51742 | -1,07 | 1,21E-07 |
| ILMN_2044617 | MTERFD1 | NM_015942 3 | 51001 | -1,08 | 9,32E-07 |
| ILMN_1754489 | FBXL20 | NM_032875 1 | 84961 | -1,08 | 1,03E-07 |
| ILMN_3229324 | SGK1 | NM_005627 3 | 6446 | -1,09 | 4,14E-07 |
| ILMN_1676588 | CEPT1 | NM_001007794 1 | 10390 | -1,09 | 1,79E-07 |
| ILMN_1743104 | RBM4B | NM_031492 2 | 83759 | -1,10 | 1,06E-07 |
| ILMN_1737580 | PPPDE2 | NM_015704 1 | 27351 | -1,10 | 5,94E-08 |
| ILMN_1735360 | SDAD1 | NM_018115 2 | 55153 | -1,10 | 5,59E-09 |
| ILMN_2234187 | CDO1 | NM_001801 2 | 1036 | -1,10 | 2,57E-07 |
| ILMN_1694847 | TERF1 | NM_017489 1 | 7013 | -1,11 | 2,75E-08 |
| ILMN_3274596 | LOC286512 | XR_038196 1 | 286512 | -1,11 | 4,69E-06 |
| ILMN_1778836 | SFRS7 | NM_001031684 1 | 6432 | -1,11 | 3,93E-07 |
| ILMN_1714700 | TRIB2 | NM_021643 1 | 28951 | -1,12 | 1,13E-06 |
| ILMN_1690921 | STAT2 | NM_005419 2 | 6773 | -1,12 | 1,31E-08 |
| ILMN_1814789 | UBAP2L | NM_014847 2 | 9898 | -1,12 | 1,31E-07 |
| ILMN_1676899 | YEATS2 | NM_018023 3 | 55689 | -1,12 | 6,87E-08 |
| ILMN_1666615 | PREPL | NM_006036 2 | 9581 | -1,13 | 1,11E-06 |
| ILMN_2223130 | SMARCA5 | NM_003601 2 | 8467 | -1,14 | 1,06E-07 |
| ILMN_1657632 | ZMYM6 | NM_007167 2 | 9204 | -1,14 | 1,04E-07 |
| ILMN_1794501 | HAS3 | NM_005329 2 | 3038 | -1,14 | 1,95E-07 |
| ILMN_1766094 | MOSPD2 | NM_152581 2 | 158747 | -1,15 | 7,33E-08 |
| ILMN_1709674 | GFPT2 | NM_005110 1 | 9945 | -1,15 | 5,02E-08 |
| ILMN_2371590 | DDX17 | NM_030881 2 | 10521 | -1,15 | 2,13E-07 |
| ILMN_1655557 | INTS6 | NM_001039937 1 | 26512 | -1,15 | 1,58E-08 |
| ILMN_2388547 | EPSTI1 | NM_033255 2 | 94240 | -1,16 | 2,19E-07 |
| ILMN_1717877 | IVNS1ABP | NM_006469 4 | 10625 | -1,16 | 1,03E-07 |
| ILMN_2356838 | CEPT1 | NM_006090 3 | 10390 | -1,18 | 4,65E-08 |
| ILMN_1892403 | SNORD13 | NR_003041 1 | 692084 | -1,19 | 1,81E-06 |
| ILMN_1676629 | INSIG2 | NM_016133 2 | 51141 | -1,20 | 2,68E-08 |
| ILMN_1734276 | PMEPA1 | NM_199169 1 | 56937 | -1,20 | 4,92E-08 |
| ILMN_1689842 | SC4MOL | NM_006745 3 | 6307 | -1,20 | 5,72E-08 |
| ILMN_1682799 | STAMBPL1 | NM_020799 2 | 57559 | -1,20 | 5,48E-09 |
| ILMN_1737298 | MAT2A | NM_005911 4 | 4144 | -1,23 | 2,82E-09 |
| ILMN_1752810 | LARP6 | NM_018357 2 | 55323 | -1,24 | 2,82E-09 |
| ILMN_1666096 | ACSL3 | NM_004457 3 | 2181 | -1,24 | 1,54E-08 |
| ILMN_1781010 | ARHGEF3 | NM_019555 1 | 50650 | -1,26 | 5,11E-09 |
| ILMN_1785852 | OBFC2A | NM_001031716 1 | 64859 | -1,27 | 2,82E-09 |
| ILMN_1803686 | ADA | NM_000022 2 | 100 | -1,27 | 5,06E-09 |
| ILMN_1688630 | RECK | NM_021111 1 | 8434 | -1,30 | 1,04E-07 |
| ILMN_1741021 | CH25H | NM_003956 3 | 9023 | -1,31 | 4,28E-09 |
| ILMN_1769615 | FLRT2 | NM_013231 4 | 23768 | -1,32 | 4,80E-10 |
| ILMN_2067269 | RECK | NM_021111 1 | 8434 | -1,35 | 2,19E-07 |
| ILMN_2054725 | SUGT1 | NM_006704 2 | 10910 | -1,36 | 1,36E-09 |
| ILMN_1738552 | SLC1A3 | NM_004172 3 | 6507 | -1,45 | 1,62E-09 |
| ILMN_1806023 | JUN | NM_002228 3 | 3725 | -1,47 | 9,01E-11 |
| ILMN_1674243 | TFRC | NM_003234 1 | 7037 | -1,48 | 4,58E-11 |
| ILMN_2187727 | NOC3L | NM_022451 9 | 64318 | -1,52 | 1,36E-09 |
| ILMN_1685722 | EIF4A2 | NM_001967 3 | 1974 | -1,58 | 9,96E-11 |
| ILMN_1687978 | PHLDA1 | NM_007350 3 | 22822 | -1,84 | 1,21E-11 |
| ILMN_1699651 | IL6 | NM_000600 1 | 3569 | -1,88 | 1,21E-11 |
| ILMN_1676663 | TNFRSF11B | NM_002546 3 | 4982 | -1,93 | 7,76E-13 |

### References

1. Haynesworth SE, Baber MA, & Caplan AI (1996) Cytokine expression by human marrow-derived mesenchymal progenitor cells in vitro: effects of dexamethasone and IL-1 alpha. J Cell Physiol 166(3):585-592.
2. Le Blanc K, et al. (2008) Mesenchymal stem cells for treatment of steroid-resistant, severe, acute graft-versus-host disease: a phase II study. The Lancet 371(9624):1579-1586.
3. Caplan AI (2009) Why are MSCs therapeutic? New data: new insight. JPathol 217(2):318-324.
4. Baker M (2009) Stem-cell drug fails crucial trials. Nature News*.*
5. Garcia-Olmo D, et al. (2009) Expanded adipose-derived stem cells for the treatment of complex perianal fistula: a phase II clinical trial. Diseases of the colon and rectum 52:79-86.
6. Menasche P (2010) Cardiac cell therapy: Lessons from clinical trials. Journal of Molecular and Cellular Cardiology*.*
7. Bartholomew A, et al. (2002) Mesenchymal stem cells suppress lymphocyte proliferation in vitro and prolong skin graft survival in vivo. Exp Hematol 30(1):42-48.
8. Chabannes D, et al. (2007) A role for heme oxygenase-1 in the immunosuppressive effect of adult rat and human mesenchymal stem cells. Blood 110(10):3691-3694.
9. Falanga V, et al. (2007) Autologous bone marrow-derived cultured mesenchymal stem cells delivered in a fibrin spray accelerate healing in murine and human cutaneous wounds. Tissue engineering 13:1299-1312.
10. Orlic D, et al. (2003) Bone marrow stem cells regenerate infarcted myocardium. Pediatric transplantation 7 Suppl 3:86-88.
11. Quevedo HC, et al. (2009) Allogeneic mesenchymal stem cells restore cardiac function in chronic ischemic cardiomyopathy via trilineage differentiating capacity. Proc Natl Acad Sci U S A 106(33):14022-14027.
12. Nagaya N (2005) Transplantation of Mesenchymal Stem Cells Improves Cardiac Function in a Rat Model of Dilated Cardiomyopathy. Circulation 112(8):1128-1135.
13. Shabbir A, Zisa D, Suzuki G, & Lee T (2009) Heart failure therapy mediated by the trophic activities of bone marrow mesenchymal stem cells: a noninvasive therapeutic regimen. Am J Physiol Heart Circ Physiol 296(6):1888-1897.
14. Uemura R, Xu M, Ahmad N, & Ashraf M (2006) Bone Marrow Stem Cells Prevent Left Ventricular Remodeling of Ischemic Heart Through Paracrine Signaling. Circ Res 98(11):1414-1421.
15. Leiker M, Suzuki G, Iyer VS, Canty Jr. JM, & Lee T (2008) Assessment of a Nuclear Affinity Labeling Method for Tracking Implanted Mesenchymal Stem Cells. Cell Transplantation 17:911-922.
16. Crisostomo P, Markel TA, Wang Y, & Meldrum D (2008) Surgically relevant aspects of stem cell paracrine effects. Surgery 143(5):577-581.
17. Lennon DP, Edmison JM, & Caplan AI (2001) Cultivation of rat marrow-derived mesenchymal stem cells in reduced oxygen tension: effects on in vitro and in vivo osteochondrogenesis. Journal of cellular physiology 187:345-355.
18. Hu X, et al. (2008) Transplantation of hypoxia-preconditioned mesenchymal stem cells improves infarcted heart function via enhanced survival of implanted cells and angiogenesis. J Thorac Cardiovasc Surg 135(4):799-808.
19. Hung S-C, Pochampally RR, Chen S-C, Hsu S-C, & Prockop DJ (2007) Angiogenic effects of human multipotent stromal cell conditioned medium activate the PI3K-Akt pathway in hypoxic endothelial cells to inhibit apoptosis, increase survival, and stimulate angiogenesis. Stem Cells 25(9):2363-2370.
20. Hung SC, et al. (2007) Short-term exposure of multipotent stromal cells to low oxygen increases their expression of CX3CR1 and CXCR4 and their engraftment in vivo. PLoS ONE 2(5):e416.
21. Lendahl U, Lee KL, Yang H, & Poellinger L (2009) Generating specificity and diversity in the transcriptional response to hypoxia. Nature reviews 10(12):821-832.
22. Volkmer E, et al. (2010) Hypoxic Preconditioning of Human Mesenchymal Stem Cells Overcomes Hypoxia-Induced Inhibition of Osteogenic Differentiation. Tissue Engineering Part A 16(1): 153-164.
23. Sammes et al (1996) Modern bioassays using metal chelates as luminescent probes. National Product Reports 13:1-28.
24. Caragounis et al (2007) Differential modulation of Alzheimer's disease amyloid beta-peptide accumulation by diverse classes of metal ligands. Biochemical Journal 407: 435-450
25. Dominici et al (2006) Minimal criteria for defining multipotent mesenchymal stromal cells. The International Society for Cellular Therapy position statement. Cytotherapy 8(4):315-317
26. Doorn J, et al. (2011) Pro-osteogenic trophic effects by PKA activation in human mesenchymal stromal cells. Biomaterials 32(26):6089-6098.
27. Siddappa R, et al. (2009) cAMP/PKA signaling inhibits osteogenic differentiation and bone formation in rodent models. Tissue Eng Part A 15(8):2135-2143.
28. Xia M, et al. (2009) Identification of Chemical Compounds that Induce HIF-1{alpha} Activity. Toxicol. Sci. 112(1):153-163.
29. Chau NM, et al. (2005) Identification of novel small molecule inhibitors of hypoxia-inducible factor-1 that differentially block hypoxia-inducible factor-1 activity and hypoxia-inducible factor-1alpha induction in response to hypoxic stress and growth factors. (Translated from eng) Cancer Research 65(11):4918-4928 (in eng).
30. Liu J, et al. (2010) In vitro and in vivo bioluminescent imaging of hypoxia in tissue-engineered grafts. (Translated from eng) Tissue engineering. Part C, Methods 16(3):479-485 (in eng).
31. Wenger RH (2005) Integration of Oxygen Signaling at the Consensus HRE. Science's STKE 2005(306):re12-re12.
32. Amin AH, et al. (2010) Modified multipotent stromal cells with epidermal growth factor restore vasculogenesis and blood flow in ischemic hind-limb of type II diabetic mice. (Translated from eng) Lab Invest 90(7):985-996 (in eng).
33. Pola R, et al. (2001) The morphogen Sonic hedgehog is an indirect angiogenic agent upregulating two families of angiogenic growth factors. (Translated from eng) Nat Med 7(6):706-711 (in eng).
34. Kusano KF, et al. (2005) Sonic hedgehog myocardial gene therapy: tissue repair through transient reconstitution of embryonic signaling. Nature Medicine 11(11):1197-1204.
35. Rey S, et al. (2009) Synergistic effect of HIF-1alpha gene therapy and HIF-1-activated bone marrow-derived angiogenic cells in a mouse model of limb ischemia. Proceedings of the National Academy of Sciences 106(48):20399-20404.
36. Kubo M, et al. (2007) Short-term pretreatment with low-dose hydrogen peroxide enhances the efficacy of bone marrow cells for therapeutic angiogenesis. (Translated from eng) American journal of physiology. Heart and circulatory physiology 292(6):H2582-2588 (in eng).
37. Kelly BD, et al. (2003) Cell Type-Specific Regulation of Angiogenic Growth Factor Gene Expression and Induction of Angiogenesis in Nonischemic Tissue by a Constitutively Active Form of Hypoxia-Inducible Factor 1. Circ Res 93(11):1074-1081.
38. Benita Y, et al. (2009) An integrative genomics approach identifies Hypoxia Inducible Factor-1 (HIF-1)-target genes that form the core response to hypoxia. (Translated from eng) Nucleic Acids Res 37(14):4587-4602 (in eng).
39. Ebert AD & Svendsen CN (2010) Human stem cells and drug screening: opportunities and challenges. Nat Rev Drug Discou 9(5):367-372.
40. Maxwell P & Salnikow K (2004) HIF-1: an oxygen and metal responsive transcription factor. (Translated from eng) Cancer Biol Ther 3(1):29-35 (in eng).
41. Murray AJ (2008) Pharmacological PKA inhibition: all may not be what it seems. (Translated from eng) Sci Signal 1(22):re4 (in eng).
42. Kim KS, Rajagopal V, Gonsalves C, Johnson C, & Kalra VK (2006) A Novel Role of Hypoxia-Inducible Factor in Cobalt Chloride- and Hypoxia-Mediated Expression of IL-8 Chemokine in Human Endothelial Cells. The Journal of Immunology 177(10):7211-7224.
43. Galindo M, et al. (2001) Hypoxia induces expression of the chemokines monocyte chemoattractant protein-1 (MCP-1) and IL-8 in human dermal fibroblasts. Clinical & Experimental Immunology 123(1):36-41.
44. Karashima T, et al. (2003) Nuclear factor-kappaB mediates angiogenesis and metastasis of human bladder cancer through the regulation of interleukin-8. (Translated from eng) Clin Cancer Res 9(7):2786-2797 (in eng).
45. Chan DA, et al. (2009) Tumor Vasculature Is Regulated by PHD2-Mediated Angiogenesis and Bone Marrow-Derived Cell Recruitment. Cancer Cell 15(6):527-538.
46. Ruas JL, et al. (2010) Complex regulation of the transactivation function of hypoxia-inducible factor-1 alpha by direct interaction with two distinct domains of the CREB-binding protein/p300. (Translated from eng) The Journal of biological chemistry 285(4):2601-2609 (in eng).
47. Di Santo S YZ, Wyler von Ballmoos M, Voelzmann J, Diehm N, Baumgartner I, Kalka C. (2009) Novel cell-free strategy for therapeutic angiogenesis: in vitro generated conditioned medium can replace progenitor cell transplantation. PLoS One 4(5):e5643.
48. Li A, Dubey S, Varney ML, Dave BJ, & Singh RK (2003) IL-8 Directly Enhanced Endothelial Cell Survival, Proliferation, and Matrix Metalloproteinases Production and Regulated Angiogenesis. The Journal of Immunology 170(6):3369-3376.
50. Fernandes H, et al. (2010) Endogenous collagen influences differentiation of human multipotent mesenchymal stromal cells. (Translated from eng) Tissue Eng Part A 16(5):1693-1702 (in eng).
51. Shibata T, Akiyama N, Noda M, Sasai K, & Hiraoka M (1998) Enhancement of gene expression under hypoxic conditions using fragments of the human vascular endothelial growth factor and the erythropoietin genes. (Translated from eng) Int J Radiat Oncol Biol Phys 42(4):913-916 (in eng).
52. O'Brien J, Wilson I, Orton T, & Pognan F (2000) Investigation of the Alamar Blue (resazurin) fluorescent dye for the assessment of mammalian cell cytotoxicity. (Translated from eng) Eur J Biochem 267(17):5421-5426 (in eng).
53. Livak KJ & Schmittgen TD (2001) Analysis of relative gene expression data using real-time quantitative PCR and the 2(-Delta Delta C(T)) method. Methods 25(4):402-408.
54. Wettenhall JM & Smyth GK (2004) limmaGUI: a graphical user interface for linear modeling of microarray data. Bioinformatics 20(18):3705-3706.
55. Langer (1983) Implantable controlled release systems. Pharmacology & Therapeutics 21(1):35-51
56. Said et al (2009) Quantifying tissue level ischemia: hypoxia response element-luciferase transfection in a rabbit ear model. Wound Repair and Regeneration 17:473― 479
57. Saha et al (2011) In vivo Bioluminescence Imaging of Tumor Hypoxia Dynamics of Breast Cancer Brain Metastasis in a Mouse Model. Journal of Visualized Experiments 3 (56). pii: 3175
58. Salvesen, GS & Nagase, H (2001) "Inhibition of proteolytic enzymes". Proteolytic enzymes: a practical approach, 2nd edition 1: 105―130
59. Pountos, I & Giannoudis PV (2005) Biology of mesenchymal stem cells. Injury, International Journal of the Care of the Injured 36S: S8 ― S12.
60. Lui, PPY & Chan KM (2011) Tendon-Derived Stem Cells (TDSCs): From Basic Science to Potential Roles in Tendon Pathology and Tissue Engineering Applications. Stem Cell Reviews, published ahead of print: DOI 10. 1007/s12015-011-9276-0.
61. Young M (2011) Stem Cell Applications in Tendon Disorders: A Clinical Perspective. Stem Cells International, published ahead of print: www.hindawi.com/journals/sci/aip/637836/

## Claims

1. A method for culturing mesenchymal stromal cell (MSC), the method comprising:
- providing a MSC; and
- culturing said MSC in the presence of 1, 10-phenanthroline or an active derivative thereof;

2. A method according to claim 1, the method further comprising harvesting the thus cultured cell(s) and/or a supernatant thereof.

3. A method according to claim 1 or 2, wherein the duration of culturing said MSC in the presence of 1, 10-phenanthroline or the active derivative thereof is for at least 12 hours.

4. A collection of cells and/or a supernatant, obtainable by a method according to claim 2 or 3.

5. A collection of cells according to claim4 and/or a supernatant according to claim 4, for use as a medicament.

6. A collection of cells and/or supernatant according to claim 4, for use in treating ischemia, for use in treating osteoporosis, for use in treating bone fractures, for inducing angiogenesis, for accelerating wound healing, for accelerating tendon regeneration, and/or for accelerating ligament regeneration.

7. Use of 1, 10-phenanthroline or an active derivative thereof for improving the angiogenic properties of a MSC and/or supernatant thereof, for inducing differentiation in a MSC, and/or for improving the angiogenic properties of a medical product.

8. Use according to claim 7, wherein said MSC is induced to differentiate into an endothelial (precursor) cell, an osteoblast (precursor) cell, a tendon precursor cell, or a ligament precursor cell.

9. Method for treating a subject, the method comprising: administering to said person a suitable amount of cells and/or supernatant according to claim 4.

10. Method according to claim 9, wherein said subject is in need of angiogenesis, in need of treatment for ischemia, in need of wound healing, in need of tendon regeneration, in need of beta cell islets and/or in need of ligament regeneration.

11. A medical product comprising 1, 10-phenanthroline and/or an active derivative thereof, cells according to claim 4, and/or supernatant according to claim 4.

12. The medical product of claim 11, wherein said medical product is a suture, a patch, a wound dressing, a pump, a plug, an implant or a graft.

13. The medical product according to claim 11 or 12 for use in replacing a missing biological structure, supporting a damaged biological structure, or enhancing an existing biological structure.

14. A method for replacing a missing biological structure, supporting a damaged biological structure, and/or enhancing an existing biological structure, the method comprising the use of cells according to claim 4, supernatant according to claim 4, 1, 10-phenanthroline or an active derivative thereof, and/or a medical product according to any one of claims 11-13, to replace, support or enhance a biological structure.
